# FASCICULE DE BREVET EUROPEEN

(11) **EP 3 902 791 B1**
(45) Date de publication et mention de la délivrance du brevet: **08.02.2023**
(21) Numéro de dépôt: 19848882.7
(22) Date de dépôt: 19.12.2019
(51) Int. Cl.: C07D 317/34, C08F 8/30, C07D 317/38

(54) **NOUVEAUX COMPOSES DIPOLAIRES AZOTES COMPRENANT DES FONCTIONS CARBONATE CYCLIQUES**
NEUARTIGE STICKSTOFFHALTIGE DIPOLVERBINDUNGEN MIT CYCLISCHEN CARBONATFUNKTIONEN
NOVEL NITROGENOUS DIPOLE COMPOUNDS COMPRISING CYCLIC CARBONATE FUNCTIONS

(30) Priorité: 27.12.2018 FR 1874233
(43) Date de publication de la demande: 03.11.2021
(73) Titulaire: COMPAGNIE GENERALE DES ETABLISSEMENTS MICHELIN, 63000 Clermont-Ferrand (FR)
(72) Inventeur: JEAN-BAPTISTE-DIT-DOMINIQUE, François, 63040 CLERMONT-FERRAND Cedex 9 (FR); IVANOV, Sergey, 63040 CLERMONT-FERRAND Cedex 9 (FR); NASYBULLIN, Ruslan, 63040 CLERMONT-FERRAND Cedex 9 (FR); UGOLNIKOV, Oleg, 63040 CLERMONT-FERRAND Cedex 9 (FR)
(74) Mandataire: Bocchi, Brigitte
(86) Numéro de dépôt international: PCT/FR2019/053188
(87) Numéro de publication internationale: WO 2020/136330

(56) Documents cités:
- WO-A1-2012/007441
- WO-A1-2012/007684
- US-B2- 7 186 845

## Description

La présente invention concerne des nouveaux composés aptes à être greffés sur un polymère, c'est-à-dire des composés comprenant une fonction capable de réagir avec un polymère comprenant initialement au moins une insaturation pour former une liaison covalente avec ledit polymère. Ces composés permettent de fonctionnaliser des polymères après polymérisation. La présente invention concerne également un procédé de préparation de ces nouveaux composés.

### Arrière-plan technique

Dans le domaine industriel, des mélanges de polymères avec des charges sont souvent utilisés. Pour que de tels mélanges présentent de bonnes propriétés, on recherche en permanence des moyens pour améliorer la dispersion des charges au sein des polymères.

En particulier pour des compositions de caoutchouc destinées à la fabrication de pneumatiques, les manufacturiers recherchent en permanence que les compositions de caoutchouc chargées possèdent de bonnes propriétés mécaniques, telles que le renforcement, et une hystérèse aussi faible que possible, synonyme d'une basse résistance au roulement.

On sait, que d'une manière générale, pour obtenir les propriétés de renforcement optimales conférées par une charge renforçante, il convient que cette dernière soit présente dans la matrice élastomèrique sous forme finale qui soit à la fois la plus finement divisée possible et répartie de la façon la plus homogène possible. Or, de telles conditions ne peuvent être réalisées que dans la mesure où la charge renforçante présente une très bonne aptitude, d'une part à s'incorporer dans la matrice élastomèrique lors du mélange avec l'élastomère et à se désagglomérer, d'autre part à se disperser de façon homogène dans cette matrice.

De manière tout à fait connue, le noir de carbone présente de telles aptitudes, ce qui n'est en général pas le cas des charges inorganiques. En effet, pour des raisons d'affinités réciproques, les particules de charge inorganique ont une fâcheuse tendance, dans la matrice élastomèrique, à s'agglomérer entre elles. Ces interactions ont pour conséquences néfastes de limiter la dispersion de la charge et donc de limiter les propriétés de renforcement à un niveau sensiblement inférieur à celui qu'il serait théoriquement possible d'atteindre si toutes les liaisons (charges renforçantes/élastomères) susceptibles d'être créées pendant l'opération de mélangeage auraient été effectivement obtenues.

De nombreuses solutions ont déjà été expérimentées pour atteindre une bonne dispersion de la charge renforçante dans une composition de caoutchouc et pour obtenir des compositions de caoutchouc présentant de bonnes propriétés de renforcement ainsi qu'une baisse de l'hystérèse.

En particulier, on peut citer la modification de la structure des polymères en fin de polymérisation au moyen d'agents de fonctionnalisation, de couplage ou d'étoilage dans le but d'obtenir une bonne interaction entre le polymère ainsi modifié et la charge renforçante, qu'il s'agisse du noir de carbone ou d'une charge inorganique renforçante. On peut citer par exemple les élastomères diéniques comportant des groupes fonctionnels comprenant une liaison carbone-étain, des groupes fonctionnels aminés tels que l'aminobenzophénone, des groupes fonctionnels silanol ou polysiloxane ayant une extrémité silanol.

En particulier, il est connu du document WO2012/007684 un composé comprenant deux groupements fonctionnels, l'un étant un dipôle azoté apte à être greffé sur un polymère comprenant au moins une insaturation et l'autre étant un groupement associatif, en particulier cycle imidazolidinone, capable d'interagir avec la charge renforçante en formant des liaisons labiles. Par « groupement associatif », on entend ici des groupes susceptibles de s'associer les uns avec les autres par des liaisons hydrogènes, ioniques et/ou hydrophobes ; ces liaisons étant des liaisons labiles. Un exemple d'un tel composé dipolaire est l'oxyde de 2-[2-(2-oxoimidazolidin-1-yl)éthoxy]benzonitrile. Il s'avère que ce composé permet une bonne interaction polymère greffé/ charge renforçante bénéfique pour les propriétés finales de la composition de caoutchouc comprenant un tel polymère greffé.

Cependant, il existe toujours un besoin constant de disposer de polymères modifiés, en particulier d'élastomères modifiés qui permettent d'obtenir des compositions de caoutchouc présentant des propriétés améliorées.

Poursuivant ses recherches, le demandeur a découvert qu'un composé dipolaire azoté comprenant fonctions carbonate cycliques qui une fois greffé à un polymère conduit à une bonne dispersion de la charge renforçante et, de manière surprenante, à d'excellentes propriétés de renforcement et d'hystérèse conférées à des compositions de caoutchouc le contenant.

### Description détaillée

L'invention a donc pour objet un composé de formule (I) 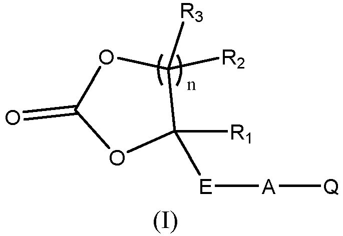 dans laquelle :
- Q représente un dipôle comprenant au moins un atome d'azote ;
- A représente un cycle arènediyle, éventuellement substitué par une ou plusieurs chaînes hydrocarbonées, identiques ou différentes, indépendantes les unes des autres, éventuellement substituées ou interrompues par un ou plusieurs hétéroatomes ;
- E représente un groupe de liaison divalent hydrocarboné pouvant éventuellement contenir un ou plusieurs hétéroatomes ;
- R₁, R₂ et R₃ représentent, indépendamment les uns des autres, un atome d'hydrogène ou une chaîne hydrocarbonée éventuellement substituée ou interrompue par un ou plusieurs hétéroatomes ; et
- n est un entier égal 1, 2, 3 ou 4.

Dans la présente, sauf indication expresse différente, tous les pourcentages (%) indiqués sont des pourcentages (%) en masse.

D'autre part, tout intervalle de valeurs désigné par l'expression « entre a et b » représente le domaine de valeurs allant de plus de a à moins de b (c'est-à-dire bornes a et b exclues) tandis que tout intervalle de valeurs désigné par l'expression « de a à b » signifie le domaine de valeurs allant de a jusqu'à b (c'est-à-dire incluant les bornes strictes a et b).

Les composés comprenant du carbone mentionnés dans la description peuvent être d'origine fossile ou biosourcée. Dans ce dernier cas, ils peuvent être, partiellement ou totalement, issus de la biomasse ou obtenus à partir de matières premières renouvelables issues de la biomasse. Sont concernés notamment les polymères, les plastifiants, les charges, etc.

L'invention ainsi que ses avantages seront aisément compris à la lumière de la description et des exemples de réalisations qui suivent.

Conformément à la formule (I), le composé selon l'invention contient un groupement Q désignant un dipôle comprenant au moins un atome d'azote.

Par « dipôle » au sens de la présente invention, on entend une fonction capable de former une addition dipolaire 1,3 sur une liaison carbone-carbone insaturée.

De préférence, le dipôle comprenant au moins un atome d'azote est choisi parmi le groupe constitué par l'oxyde de nitrile, la nitrone et la nitrile imine.

Par oxyde de nitrile, on entend au sens de la présente invention un dipôle répondant à la formule -C≡N→O, y compris ses formes mésomères.

Par imine de nitrile, on entend au sens de la présente invention un dipôle répondant à la formule -C≡→N, y compris ses formes mésomères.

Par nitrone, on entend au sens de la présente invention un dipôle répondant à la formule -C=N(->O)-, y compris ses formes mésomères.

Plus préférentiellement encore, le groupement Q est un groupement de formule (II), (III) ou (IV) 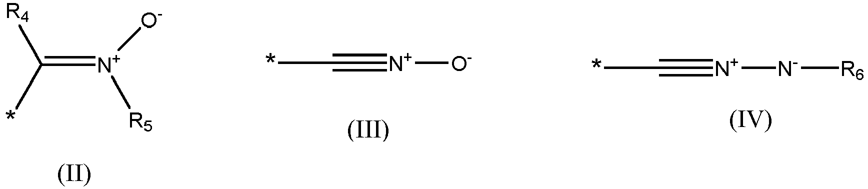 dans lesquelles :
- le symbole * représente le rattachement de Q à A ; et
- R₄, R₅ et R₆ sont choisis indépendamment parmi un atome d'hydrogène, un alkyle, linéaire ou ramifié, en C₁-C₂₀, un cycloalkyle en C₃-C₃₀ éventuellement substitué par une chaîne hydrocarbonée, un aryle en C₆-C₂₀ éventuellement substitué par une chaîne hydrocarbonée.

Par « chaîne hydrocarbonée », on entend une chaîne comprenant un ou plusieurs atomes de carbone et un ou plusieurs atomes d'hydrogène. La chaîne hydrocarbonée peut être saturée ou insaturée, de préférence saturée, linéaire, ramifiée ou cyclique et peut comprendre de 1 à 24 atomes de carbone.

De préférence R₄, R₅ et R₆ sont choisis indépendamment parmi un atome d'hydrogène, un alkyle, linéaire ou ramifié, en C₁-C₂₀, un cycloalkyle en C₃-C₃₀ éventuellement substitué par une chaîne hydrocarbonée saturée en C₁-C₂₄, un aryle en C₆-C₂₀ éventuellement substitué par une chaîne hydrocarbonée saturée en C₁-C₂₄. Plus préférentiellement encore, R₄, R₅ et R₆ sont choisis, indépendamment les uns par rapport aux autres, parmi un atome d'hydrogène, un alkyle, linéaire ou ramifié, en C₁-C₂₀, un cycloalkyle en C₃-C₃₀ éventuellement substitué par un alkyle, linéaire ou ramifié, en C₁-C₆, un aryle en C₆-C₂₀ éventuellement substitué par un alkyle, linéaire ou ramifié, en C₁-C₆.

Conformément à la formule (I), A représente un cycle arènediyle, éventuellement substitué par une ou plusieurs chaînes hydrocarbonées, identiques ou différentes, indépendantes les unes des autres, éventuellement substituées ou interrompues par un ou plusieurs hétéroatomes.

On entend au sens de la présente invention par « cycle arènediyle », un groupe hydrocarboné aromatique monocyclique ou polycyclique, dérivé d'un arène dans lequel 2 atomes d'hydrogène ont été supprimés. Un cycle arènediyle est donc un groupe divalent.

Par « groupe hydrocarboné aromatique monocyclique ou polycyclique », on entend un ou des cycles aromatiques dont le squelette est constitué d'atomes de carbone. Autrement dit, il n'y a pas d'hétéroatomes dans le squelette du cycle. Le cycle arènediyle peut être monocyclique, c'est-à-dire constitué d'un seul cycle, ou polycyclique, c'est-à-dire constitué de plusieurs cycles hydrocarbures aromatiques condensés ; de tels cycles condensés ont alors en commun au moins deux atomes de carbone successifs. Ces cycles peuvent être ortho-condensés ou ortho- et péri-condensés. De préférence, le cycle arènediyle comprend entre 6 et 14 atomes de carbone.

De préférence, lorsque le cycle arènediyle est substitué par une ou plusieurs chaîne(s) hydrocarbonée(s), identique(s) ou différente(s), indépendante(s) les unes des autres, éventuellement substituée(s) ou interrompue(s) par un ou plusieurs hétéroatome(s), cette ou ces chaîne(s) sont inertes vis-à-vis de la fonction carbonate cyclique et du groupement Q.

On entend, au sens de la présente invention, par « chaîne(s) hydrocarbonée(s) inerte(s) vis-à-vis de la fonction carbonate cyclique et du groupement Q » une chaîne hydrocarbonée qui ne réagit ni avec ladite fonction carbonate cyclique ni avec ledit groupement Q. Ainsi, ladite chaîne hydrocarbonée inerte par rapport à ladite fonction et audit groupement est, par exemple, une chaîne hydrocarbonée qui ne présente pas de fonctions alcényle ou alcynyle, susceptibles de réagir avec ladite fonction ou ledit groupement. De manière préférée, ces chaînes hydrocarbonées sont saturées et peuvent comprendre de 1 à 24 atomes de carbone.

De préférence, le groupement A est un cycle arènediyle en C₆-C₁₄ éventuellement substitué par une ou plusieurs chaînes hydrocarbonées, identiques ou différentes, indépendantes les unes des autres, éventuellement substituées ou interrompues par un ou plusieurs hétéroatomes. Plus préférentiellement, le groupement A est un cycle arènediyle, de préférence en C₆-C₁₄, éventuellement substitué par une ou plusieurs chaîne(s) hydrocarbonée(s), identique(s) ou différente(s), saturée(s) en C₁-C₂₄, éventuellement substituée(s) ou interrompue(s) par un ou plusieurs hétéroatome(s) d'azote, de soufre ou d'oxygène. Plus préférentiellement encore, le groupement A est un cycle arènediyle en C₆-C₁₄, éventuellement substitué par un ou plusieurs groupe(s) alkyle, identique(s) ou différent(s), en C₁-C₁₂, (plus préférentiellement en C₁-C₆, plus préférentiellement encore en C₁-C₄) ou par un groupe choisi par -OR', -NHR', -SR', R' étant un groupe alkyle, préférentiellement un groupe alkyle en C₁-C₁₂, plus préférentiellement en C₁-C₆, plus préférentiellement encore en C₁-C₄.

De préférence, le composé de formule (I) selon l'invention est choisi dans le groupe formé par les composés de formule (Ia) et (Ib) suivantes : 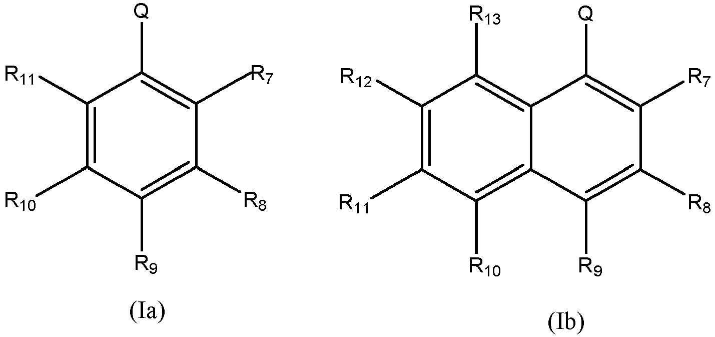 dans lesquelles :
- le groupement Q est tel que défini précédemment ; préférentiellement est choisi parmi le groupe constitué par l'oxyde de nitrile, la nitrone et la nitrile imine, plus préférentiellement Q est le groupe de formule (III);
- un groupement choisi parmi R₇ à R₁₁ de la formule (Ia) et un groupement choisi parmi R₇ à R₁₃ de la formule (Ib) désigne le groupe de formule (V) suivante : 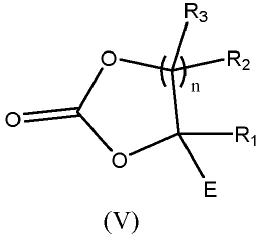 dans laquelle n, E, R₁, R₂ et R₃ sont tels que définis précédemment, et
- les quatre autres groupements de la formule (Ia) et les six autres groupements de la formule (Ib), identiques ou différents, représentent indépendamment les uns les autres, un atome d'hydrogène ou une chaîne hydrocarbonée, linéaire ou ramifiée, de préférence saturée éventuellement substituée ou interrompue par un ou plusieurs hétéroatomes.

Préférentiellement, ladite chaîne hydrocarbonée dans les composés de formule (Ia) et (Ib) est inerte vis-à-vis du groupe de formule (V) et du groupement Q. Préférentiellement, ladite chaîne hydrocarbonée est saturée et peut comprendre de 1 à 24 atomes de carbone. Préférentiellement, ladite chaîne hydrocarbonée est un alkyle en C₁-C₁₂ (plus préférentiellement en C₁-C₆, plus préférentiellement encore en C₁-C₄) ou un groupe choisi parmi -OR', -NHR', -SR', R' étant un alkyle en C₁-C₁₂, plus préférentiellement en C₁-C₆, plus préférentiellement encore en C₁-C₄.

Selon un mode de réalisation préférée de l'invention, dans la formule (Ia), R₈ représente un groupe de formule (V) tel que défini ci-dessus et R₇, R₉, R₁₀ et R₁₁, identiques ou différents, représentent un atome d'hydrogène ou une chaîne hydrocarbonée, linéaire ou ramifiée, de préférence saturée en C₁-C₂₄, éventuellement substituée ou interrompue par un ou plusieurs hétéroatomes. Plus préférentiellement, R₈ représente un groupe de formule (V) tel que défini ci-dessus et R₇, R₉, R₁₀ et R₁₁, identiques ou différents représentent un atome d'hydrogène ou un alkyle en C₁-C₁₂ (plus préférentiellement en C₁-C₆, plus préférentiellement encore en C₁-C₄) ou un groupe choisi parmi -OR', -NHR', -SR', R' étant un alkyle en C₁-C₁₂, plus préférentiellement en C₁-C₆, plus préférentiellement encore en C₁-C₄.

Plus préférentiellement encore dans ce mode de réalisation, R₈ représente un groupe de formule (V) tel que défini ci-dessus, R₁₀ représente un atome d'hydrogène et R₇, R₉ et R₁₁ représentent une chaîne hydrocarbonée, linéaire ou ramifiée, de préférence saturée en C₁-C₂₄, éventuellement substituée ou interrompue par un ou plusieurs hétéroatomes. Plus préférentiellement encore, R₈ représente un groupe de formule (V) tel que défini ci-dessus, R₁₀ représente un atome d'hydrogène et R₇, R₉ et R₁₁ représentent un alkyle en C₁-C₁₂ (plus préférentiellement en C₁-C₆, plus préférentiellement encore en C₁-C₄) ou un groupe choisi parmi -OR', -NHR', -SR', R' étant un alkyle en C₁-C₁₂, plus préférentiellement en C₁-C₆, plus préférentiellement encore en C₁-C₄.

Selon un autre mode de réalisation préférée de l'invention, dans la formule (Ib), R₇ représente un groupe de formule (V) tel que défini ci-dessus et R₈ à R₁₃ identiques ou différents, représentent un atome d'hydrogène ou une chaîne hydrocarbonée, linéaire ou ramifiée, de préférence saturée en C₁-C₂₄, éventuellement substituée ou interrompue par un ou plusieurs hétéroatomes. Plus préférentiellement, R₇ représente un groupe de formule (V) tel que défini ci-dessus et R₈ à R₁₃, identiques ou différents, représentent un atome d'hydrogène ou un alkyle en C₁-C₁₂ (plus préférentiellement en C₁-C₆, plus préférentiellement encore en C₁-C₄) ou un groupe choisi parmi -OR', -NHR', -SR', R' étant un alkyle en C₁-C₁₂, plus préférentiellement en C₁-C₆, plus préférentiellement encore en C₁-C₄. Plus préférentiellement encore dans ce mode de réalisation, R₇ représente un groupe de formule (V) tel que défini ci-dessus et R₈ à R₁₃, identiques, représentent un atome d'hydrogène.

Conformément aux composés de formule (I), (Ia) et (Ib), le groupement E est un groupe de liaison divalent hydrocarboné pouvant éventuellement contenir un ou plusieurs hétéroatome(s). Par « groupe de liaison divalent hydrocarboné » on entend au sens de la présente invention, un groupe espaceur formant un pont entre le groupement A et le groupe de formule (V), ce groupe espaceur étant une chaîne hydrocarbonée, saturée ou insaturée, de préférence saturée en C₁-C₂₄, linéaire ou ramifiée, pouvant éventuellement contenir un ou plusieurs hétéroatome(s) tel(s) que par exemple N, O et S. Ladite chaîne hydrocarbonée peut éventuellement être substituée, pour autant que les substituants ne réagissent pas avec le groupement Q et le groupe de formule (V) tel que défini ci-dessus.

Préférentiellement, dans les composés de formule (I), (Ia) et (Ib), le groupement E est une chaîne hydrocarbonée linéaire ou ramifiée, de préférence saturée en C₁-C₂₄, plus préférentiellement en C₁-C₁₀, encore plus préférentiellement en C₁-C₆, éventuellement interrompue par un ou plusieurs atomes d'azote, de soufre ou d'oxygène.

De préférence, dans les composés de formule (I), (Ia) et (Ib), le groupement E est choisi dans le groupe constitué par -R-, -NH-R-, -O-R- et -S-R- avec R un alkylène linéaire ou ramifié en C₁-C₂₄, de préférence en C₁-C₁₀, plus préférentiellement en C₁-C₆.

Plus préférentiellement encore, dans les composés de formule (I), (Ia) et (Ib), le groupement E est choisi dans le groupe constitué par -R- et -O-R- avec R un alkylène linéaire ou ramifié en C₁-C₂₄, de préférence en C₁-C₁₀, plus préférentiellement en C₁-C₆.

Plus préférentiellement encore, dans les composés de formule (I), (Ia) et (Ib), le groupement E est choisi parmi -CH₂-, -CH₂-CH₂-, -CH₂-CH₂-CH₂-, -CH₂-CH₂-CH₂-CH₂-, -O-CH₂-, -O-CH₂-CH₂-, -O-CH₂-CH₂-CH₂- et -O-CH₂-CH₂-CH₂- CH₂-.

Dans les composés de formule (I), (Ia) et (Ib), n est un entier ayant pour valeur 1, 2, 3 ou 4 ; plus préférentiellement n est un entier ayant pour valeur 1 ou 2, encore plus préférentiellement n = 1.

Dans les composés de formule (I), (Ia) et (Ib), R₁, R₂ et R₃ représentent, indépendamment les uns des autres, un atome d'hydrogène ou une chaîne hydrocarbonée éventuellement substituée ou interrompue par un ou plusieurs hétéroatome(s), tel(s) que par exemple N, O et S. Plus préférentiellement, les groupes R₁, R₂, R₃ représentent, indépendamment les uns des autres, un atome d'hydrogène ou un alkyle linéaire ou ramifié en C₁-C₂₄, préférentiellement en C₁-C₁₀, plus préférentiellement en C₁-C₆. Plus préférentiellement encore, le groupe R₁ est un atome d'hydrogène et les groupes R₂ et R₃, identiques ou différents, sont des alkyles linéaires ou ramifiés en C₁-C₂₄, préférentiellement en C₁-C₁₀, plus préférentiellement en C₁-C₆. Plus préférentiellement encore, R₁, R₂ et R₃, identiques, sont un atome d'hydrogène. Préférentiellement, parmi les composés de formule (I), les composés de formule (VI) sont particulièrement préférés 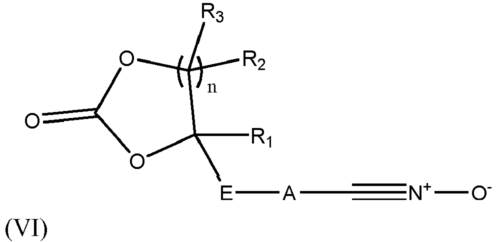 dans laquelle :
- A représente un cycle arènediyle, éventuellement substitué par une ou plusieurs chaînes hydrocarbonées, identiques ou différentes, indépendantes les unes des autres, éventuellement substituées ou interrompues par un ou plusieurs hétéroatomes ;
- E représente un groupe divalent hydrocarboné pouvant éventuellement contenir un ou plusieurs hétéroatomes ;
- R₁, R₂ et R₃ représentent, indépendamment les uns des autres, un atome d'hydrogène ou une chaîne hydrocarbonée éventuellement substituée ou interrompue par un ou plusieurs hétéroatomes ; et
- n est un entier égal à 1, 2, 3 ou 4.

De préférence, dans les composés de formule (VI), le groupement A est un cycle arènediyle de préférence en C₆-C₁₄ éventuellement substitué par une ou plusieurs chaîne(s) hydrocarbonée(s), identique(s) ou différente(s), indépendante(s) les unes des autres, de préférence saturée(s) en C₁-C₂₄, éventuellement substituée(s) ou interrompue(s) par un ou plusieurs hétéroatome(s) par exemple, tel(s) que O, N et S. Plus préférentiellement, le groupement A est un cycle arènediyle en C₆-C₁₄ éventuellement substitué par un ou plusieurs groupe(s) alkyle, identique(s) ou différent(s), en C₁-C₁₂ (plus préférentiellement en C₁-C₆, plus préférentiellement encore en C₁-C₄) ou par un groupe choisi par -OR', -NHR', -SR', R' étant un groupe alkyle en C₁-C₁₂, plus préférentiellement en C₁-C₆, plus préférentiellement encore en C₁-C₄.

Plus préférentiellement encore, parmi les composés de formule (VI), les composés de formule (VIa) et (VIb) sont particulièrement préférés 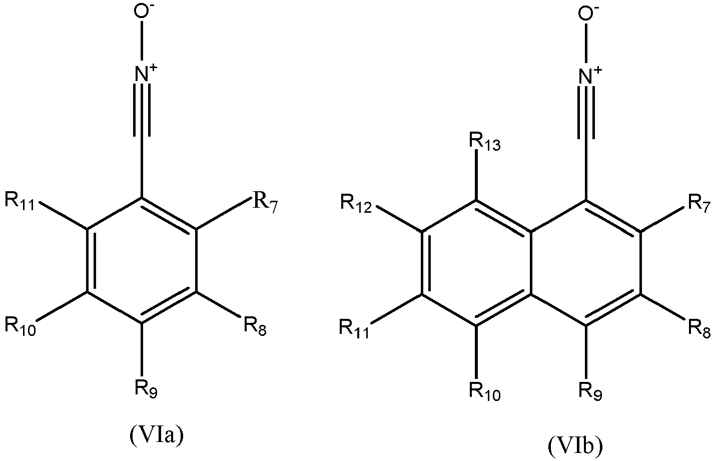 dans lesquelles :
- un groupement choisi parmi R₇ à R_{11\} de la formule (VIa) et un groupement choisi parmi R₇ à R₁₃ de la formule (VIb) désigne le groupe de formule (V) suivante : 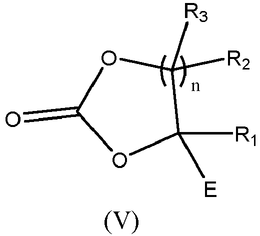 dans laquelle n, E, R₁, R₂ et R₃ sont tels que définis précédemment, et
- les quatre autres groupements de la formule (VIa) et les six autres groupements de la formule (VIb), identiques ou différents, représentent indépendamment les uns les autres, un atome d'hydrogène ou une chaîne hydrocarbonée, linéaire ou ramifiée, de préférence saturée en C₁-C₂₄, éventuellement substituée ou interrompue par un ou plusieurs hétéroatomes. Préférentiellement, ladite chaîne hydrocarbonée dans les composés de formule (VIa) et (VIb) est inerte vis-à-vis du groupe de formule (V) et du groupement Q. Préférentiellement, ladite chaîne hydrocarbonée est un alkyle en C₁-C₁₂ (plus préférentiellement en C₁-C₆, plus préférentiellement encore en C₁-C₄) ou un groupe choisi parmi -OR', -NHR', -SR', R' étant un alkyle en C₁-C₁₂, plus préférentiellement en C₁-C₆, plus préférentiellement encore en C₁-C₄.

Préférentiellement, dans les composés de formule (VI), (VIa) et (VIb), le groupement E est une chaîne hydrocarbonée linéaire ou ramifiée, de préférence saturée en C₁-C₂₄, plus préférentiellement en C₁-C₁₀, encore plus préférentiellement en C₁-C₆, éventuellement interrompue par un ou plusieurs atomes d'azote, de soufre ou d'oxygène. De préférence, le groupement E est choisi dans le groupe constitué par -R-, -NHR-, -OR- et -SR- avec R un alkylène linéaire ou ramifié en C₁-C₂₄, de préférence en C₁-C₁₀, plus préférentiellement en C₁-C₆. Plus préférentiellement encore, le groupement E est choisi dans le groupe constitué par -R- et -O-R- avec R un alkylène linéaire ou ramifié en C₁-C₂₄, de préférence en C₁-C₁₀, plus préférentiellement en C₁-C₆. Plus préférentiellement encore, le groupement E est choisi parmi -CH₂-, -CH₂-CH₂-, -CH₂-CH₂-CH₂-, -CH₂-CH₂-CH₂-CH₂-, -O-CH₂-, -O-CH₂-CH₂-, -O-CH₂-CH₂-CH₂- et -O-CH₂-CH₂-CH₂- CH₂-.

Préférentiellement, dans les composés de formule (VI), (VIa) et (VIb), n est un entier ayant pour valeur 1, 2, 3 ou 4 ; plus préférentiellement n est un entier ayant pour valeur 1 ou 2, encore plus préférentiellement n= 1.

Préférentiellement, dans les composés de formule (VI), (VIa) et (VIb), R₁, R₂ et R₃ représentent, indépendamment les uns des autres, un atome d'hydrogène ou une chaîne hydrocarbonée éventuellement substituée ou interrompue par un ou plusieurs hétéroatome(s), tel(s) que par exemple N, O et S. Plus préférentiellement, les groupes R₁, R₂, R₃ représentent, indépendamment les uns des autres, un atome d'hydrogène ou un alkyle linéaire ou ramifié en C₁-C₂₄, préférentiellement en C₁-C₁₀, plus préférentiellement en C₁-C₆. Plus préférentiellement encore, le groupe R₁ est un atome d'hydrogène et les groupes R₂ et R₃, identiques ou différents, sont des alkyles linéaires ou ramifiés en C₁-C₂₄, préférentiellement en C₁-C₁₀, plus préférentiellement en C₁-C₆. Plus préférentiellement encore, R₁, R₂ et R₃, identiques, sont un atome d'hydrogène.

Selon un mode de réalisation préférée de l'invention, dans la formule (VIa), R₈ représente un groupe de formule (V) tel que défini ci-dessus et R₇, R₉, R₁₀ et R₁₁, identiques ou différents, représentent un atome d'hydrogène, un alkyle en C₁-C₁₂ (plus préférentiellement en C₁-C₆, plus préférentiellement encore en C₁-C₄) ou un groupe choisi parmi -OR', -NHR', -SR', R' étant un alkyle en C₁-C₁₂, plus préférentiellement en C₁-C₆, plus préférentiellement encore en C₁-C₄.

Plus préférentiellement encore dans ce mode de réalisation, R₈ représente un groupe de formule (V) tel que défini ci-dessus, R₁₀ représente un atome d'hydrogène et R₇, R₉ et R₁₁ représentent un alkyle en C₁-C₁₂ (plus préférentiellement en C₁-C₆, plus préférentiellement encore en C₁-C₄) ou un groupe choisi parmi -OR', -NHR', -SR', R' étant un alkyle en C₁-C₁₂, plus préférentiellement en C₁-C₆, plus préférentiellement encore en C₁-C₄.

Parmi les composés de formule (VIa), ceux qui sont particulièrement préférés sont ceux ayant les caractéristiques suivantes :
- R₇, R₉ et R₁₁, identiques ou différents, représentent un alkyle en C₁-C₁₂ (plus préférentiellement en C₁-C₆, plus préférentiellement encore en C₁-C₄) ou un groupe choisi parmi -OR', -NHR', -SR', R' étant un alkyle en C₁-C₁₂, plus préférentiellement en C₁-C₆, plus préférentiellement encore en C₁-C₄ ; et
- R₁₀ représente un atome d'hydrogène ; et
- R₈ représente un groupe de formule (V) avec n=1 ou 2, de préférence n=1, le groupement E est choisi dans le groupe constitué par -R- et -O-R- avec R un alkylène linéaire ou ramifié en C₁-C₂₄, de préférence en C₁-C₁₀, plus préférentiellement en C₁-C₆, plus préférentiellement encore, le groupement E est choisi parmi -CH₂-, -CH₂-CH₂-, -CH₂-CH₂-CH₂-, -CH₂-CH₂-CH₂-CH₂-, -O-CH₂-, -O-CH₂-CH₂-, -O-CH₂-CH₂-CH₂- et -O-CH₂-CH₂-CH₂-CH₂- et les groupes R₁, R₂, R₃ représentent, indépendamment les uns des autres, un atome d'hydrogène ou un alkyle linéaire ou ramifié en C₁-C₂₄, préférentiellement en C₁-C₁₀, plus préférentiellement en C₁-C₆, de préférence sont tous identiques et sont un atome d'hydrogène.

Selon un autre mode de réalisation préférée de l'invention, dans la formule (VIb), R₇ représente un groupe de formule (V) tel que défini ci-dessus et R₈ à R₁₃, identiques ou différents, représentent un atome d'hydrogène, un alkyle en C₁-C₁₂ (plus préférentiellement en C₁-C₆, plus préférentiellement encore en C₁-C₄) ou un groupe choisi parmi -OR', -NHR', -SR', R' étant un alkyle en C₁-C₁₂, plus préférentiellement en C₁-C₆, plus préférentiellement encore en C₁-C₄. Plus préférentiellement encore dans ce mode de réalisation, R₇ représente un groupe de formule (V) et R₈ à R₁₃, identiques, représentent un atome d'hydrogène.

Parmi les composés de formule (VIb), ceux qui sont particulièrement préférés sont ceux ayant les caractéristiques suivantes :
- R₇ représente un groupe de formule (V) avec n=1 ou 2, de préférence n=1, le groupement E est choisi dans le groupe constitué par -R- et -O-R- avec R un alkylène linéaire ou ramifié en C₁-C₂₄, de préférence en C₁-C₁₀, plus préférentiellement en C₁-C₆, plus préférentiellement encore, le groupement E est choisi parmi -CH₂-, -CH₂-CH₂-, -CH₂-CH₂-CH₂-, -CH₂-CH₂-CH₂-CH₂-, -O-CH₂-, -O-CH₂-CH₂-, -O-CH₂-CH₂-CH₂- et -O-CH₂-CH₂-CH₂-CH₂- et les groupes R₁, R₂, R₃ représentent, indépendamment les uns des autres, un atome d'hydrogène ou un alkyle linéaire ou ramifié en C₁-C₂₄, préférentiellement en C₁-C₁₀, plus préférentiellement en C₁-C₆, de préférence sont tous identiques et sont un atome d'hydrogène ; et
- et R₈ à R₁₃, identiques ou différents, représentent un atome d'hydrogène, un alkyle en C₁-C₁₂ (plus préférentiellement en C₁-C₆, plus préférentiellement encore en C₁-C₄) ou un groupe choisi parmi -OR', -NHR', -SR', R' étant un alkyle en C₁-C₁₂, plus préférentiellement en C₁-C₆, plus préférentiellement encore en C₁-C₄; plus préférentiellement R₈ à R₁₃, identiques, représentent un atome d'hydrogène.

Selon un mode de réalisation particulier, le composé de formule (I) selon l'invention, de préférence le composé de formule (VI), est choisi dans le groupe constitué par le composé de formule (VII) et le composé de formule (VIII)

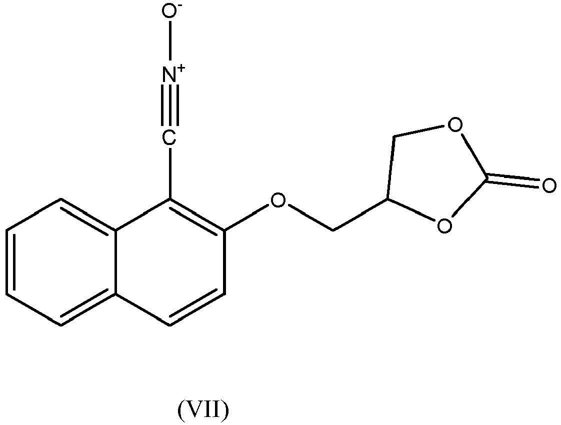

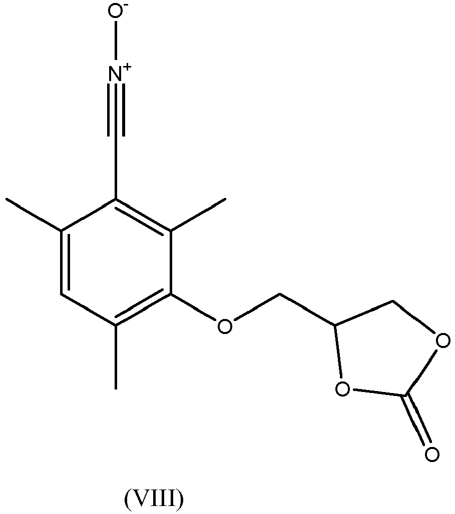

L'invention a également pour objet un procédé de préparation d'un composé de formule (VI) comprenant au moins une réaction (d) d'un composé oxime de formule (IX) avec un agent oxydant en présence d'au moins un solvant organique SL1 selon le schéma réactionnel suivant : 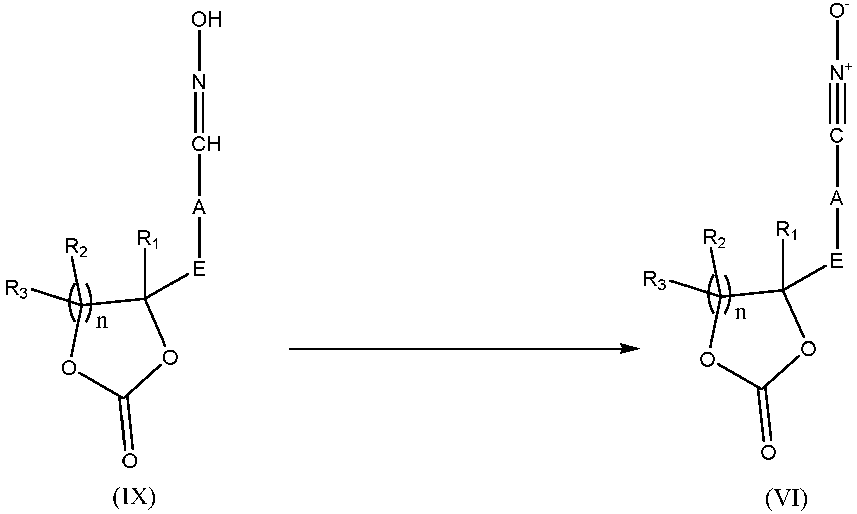 avec
- A représente un cycle arènediyle, éventuellement substitué par une ou plusieurs chaînes carbonées, identiques ou différentes, indépendantes les unes des autres, éventuellement substituées ou interrompues par un ou plusieurs hétéroatomes ;
- E représente un groupe divalent hydrocarboné pouvant éventuellement contenir un ou plusieurs hétéroatomes ;
- R₁, R₂ et R₃ représentent, indépendamment les uns des autres, un atome d'hydrogène ou une chaîne hydrocarbonée éventuellement substituée ou interrompue par un ou plusieurs hétéroatomes ; et
- n est un entier égal à 1, 2, 3 ou4.

Les modes préférés de A, E, R₁, R₂ et R₃ et n tels que décrit-ci dessus s'appliquent également au procédé de préparation d'un composé de formule (VI) à partir d'un composé de formule (IX).

De manière préférée, ledit agent oxydant est choisi parmi l'hypochlorite de sodium, le N-bromosuccinimide en présence d'une base, le N-chlorosucinimide en présence d'une base, et l'eau oxygénée en présence d'un catalyseur. Plus préférentiellement, le catalyseur est choisi parmi le groupe constitué par l'hypochlorite de sodium et le N-chlorosucinimide en présence d'une base.

Avantageusement, la quantité d'agent oxydant est de 1 à 5 équivalent molaires, préférentiellement de 1 à 2 équivalents molaires par rapport à la quantité molaire du composé oxime de formule (IX).

Préférentiellement le solvant organique SL1 est choisi parmi les solvants chlorés et les solvants de type ester, éther et alcool, plus préférentiellement choisi parmi le dichlorométhane, le trichlorométhane, l'acétate d'éthyle, l'acétate de butyle, l'éther diéthylique, l'isopropanol et l'éthanol, encore plus préférentiellement est choisi parmi l'acétate d'éthyle, le trichlorométhane, le dichlorométhane et l'acétate de butyle.

De préférence, le composé oxime de formule (IX) représente de 1 à 30 % en poids, de préférence de 1 à 20 % en poids, par rapport au poids total de l'ensemble comprenant ledit composé oxime de formule (IX), ledit solvant organique SL1 et ledit agent oxydant. L'invention a également pour objet un procédé de préparation d'un composé oxime de formule (IX) comprenant au moins une réaction (c) d'un composé de formule (X) avec une solution aqueuse d'hydroxylamine NH₂OH (composé de formule (XI)) selon le schéma réactionnel suivant : 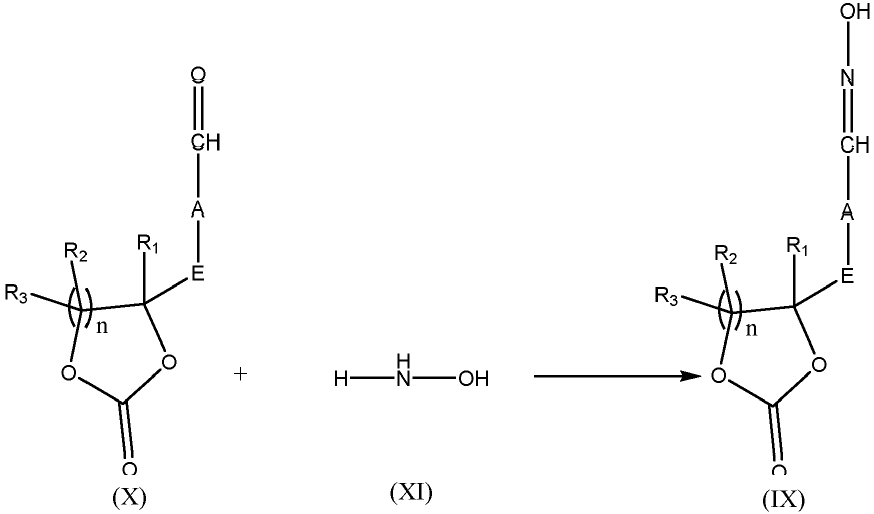 avec :
- A représente un cycle arènediyle, éventuellement substitué par une ou plusieurs chaînes carbonées, identiques ou différentes, indépendantes les unes des autres, éventuellement substituées ou interrompues par un ou plusieurs hétéroatomes ;
- E représente un groupe divalent hydrocarboné pouvant éventuellement contenir un ou plusieurs hétéroatomes ;
- R₁, R₂ et R₃ représentent, indépendamment les uns des autres, un atome d'hydrogène ou une chaîne hydrocarbonée éventuellement substituée ou interrompue par un ou plusieurs hétéroatomes ; et
- n est un entier égal à 1, 2, 3 ou 4.

Les modes préférés de A, E, R₁, R₂ et R₃ et n s'appliquent également au procédé de préparation d'un composé de formule (IX) à partir d'un composé de formule (X). Préférentiellement, l'ajout d'hydroxylamine (composé de formule (XI)) est réalisé à une température allant de 1°C à 100°C, plus préférentiellement entre 20°C et 70°C.

Préférentiellement, l'ajout de la solution aqueuse d'hydroxylamine nécessaire à la réaction décrite ci-dessus est effectué en deux fois.

Plus préférentiellement, le composé de formule (X) est mis en contact avec une première quantité de composé de formule (XI) comprise dans un domaine allant de 1,02 à 2 équivalents molaires par rapport au composé de formule (X), préférentiellement comprise dans un domaine allant de 1,1 et 1,75 équivalents molaires; puis 2 à 10 heures après cette mise en contact, une deuxième quantité de composé de formule (XI) est ajouté au milieu réactionnel. Cette deuxième quantité de composé de formule (XI) est préférentiellement comprise dans un domaine allant de 0,25 à 1,5 équivalents molaire par rapport au composé de formule (X), préférentiellement entre 0,25 et 0,75 équivalents molaires.

La réaction décrite ci-dessus peut être adaptée pour obtenir les composés de formule (I) selon l'invention à partir du composé de formule (IX). En particulier, le procédé de préparation du composé de formule (I) dans lequel Q est une nitrone comprend au moins une réaction du composé de formule (X) avec une hydroxylamine de formule NR₄R₅-OH où R₄ et R₅, identiques ou différents, de préférence différents sont tels que définis précédemment, y compris leurs modes préférés.

Le composé de formule (X) peut être obtenu par un procédé de préparation comprenant au moins une réaction (b) de carbonatation du composé de formule (XII) en présence de CO₂, d'un solvant organique SL2 et d'un catalyseur selon le schéma réactionnel suivant : 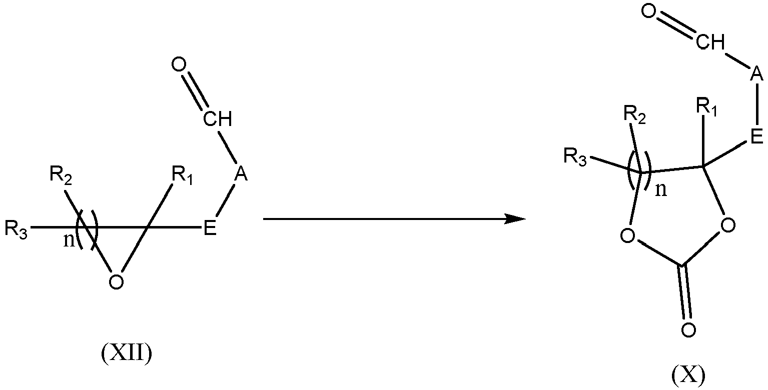 avec :
- A représente un cycle arènediyle, éventuellement substitué par une ou plusieurs chaînes carbonées, identiques ou différentes, indépendantes les unes des autres, éventuellement substituées ou interrompues par un ou plusieurs hétéroatomes ;
- E représente un groupe divalent hydrocarboné pouvant éventuellement contenir un ou plusieurs hétéroatomes ;
- R₁, R₂ et R₃ représentent, indépendamment les uns des autres, un atome d'hydrogène ou une chaîne hydrocarbonée éventuellement substituée ou interrompue par un ou plusieurs hétéroatomes ; et
- n est un entier égal à 1, 2, 3 ou 4.

Les modes préférés de A, E, R₁, R₂ et R₃ et n s'appliquent également au procédé de préparation d'un composé de formule (X) à partir d'un composé de formule (XII).

Le catalyseur peut être choisi dans le groupe constitué par les sels d'ammonium, les sels des métaux alcalino-terreux (comme par exemple les sels de zinc, les sels de cobalt), les sels des métaux pauvres (tels que les sels d'aluminium, les sels de titane, les sels d'étain). De préférence le catalyseur est un sel d'ammonium, plus préférentiellement est choisi dans le groupe constitué par le tétrabuylammonium (TBAB) et le bromure de tétrabutylammonium.

Le solvant organique SL2 est choisi parmi les solvant chlorés et les solvants de type ester, éther, alcool, amide, plus préférentiellement est choisi parmi le dichlorométhane, le trichlorométhane, l'acétate d'éthyle, l'acétate de butyle, l'éther diéthylique, l'isopropanol et l'éthanol, le N,N-diméthylformamide (DMF), le 1,4-dioxane ; encore plus préférentiellement est choisi parmi le DMF et le 1,4-dioxane.

Le composé de formule (XII) peut être obtenu par un procédé de préparation comprenant au moins une réaction (a) du composé de formule (XIII) avec un composé de formule (XIV) en présence d'au moins une base et à une température allant de 20°C à 150°C selon le schéma réactionnel suivant : 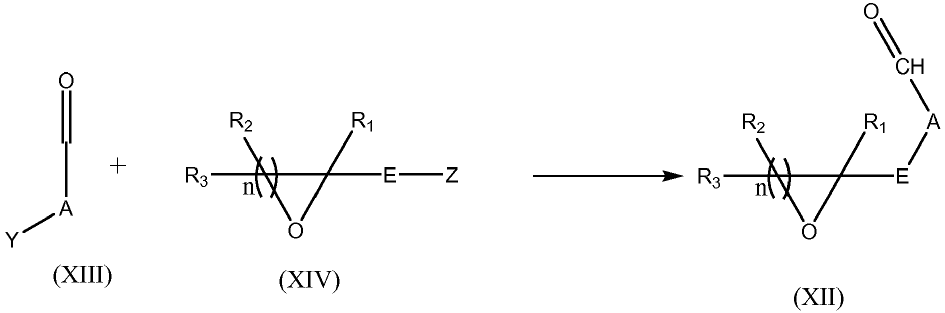 avec
- A représente un cycle arènediyle, éventuellement substitué par une ou plusieurs chaînes carbonées, identiques ou différentes, indépendantes les unes des autres, éventuellement substituées ou interrompues par un ou plusieurs hétéroatomes ;
- E représente un groupe divalent hydrocarboné pouvant éventuellement contenir un ou plusieurs hétéroatomes ;
- R₁, R₂ et R₃ représentent, indépendamment les uns des autres, un atome d'hydrogène ou une chaîne hydrocarbonée éventuellement substituée ou interrompue par un ou plusieurs hétéroatomes ;
- n est un entier égal à 1, 2, 3 ou 4 ;
- Y représente un groupe nucléophile ; et
- Z représente un groupe nucléofuge.

Les modes préférés de A, E, R₁, R₂ et R₃ et n s'appliquent également au procédé de préparation d'un composé de formule (XII) à partir des composés de formule (XIII) et (XIV).

On entend par « groupe nucléofuge » un groupe partant.

On entend « par groupe nucléophile » un composé comprenant au moins un atome porteur d'un doublet libre ou d'un atome chargé négativement.

De manière préférée, le groupe Y est choisi parmi les fonctions hydroxyle, thiol, amine primaire et amine secondaire.

Le groupe Z peut être choisi parmi le chlore, le brome, l'iode, le fluor, le groupe mésylate, le groupe tosylate, le groupe acétate, et le groupe trifluorométhylsulfonate.

Plus préférentiellement, le groupe Y est la fonction hydroxyle et le groupe Z est le chlore.

La réaction entre le composé de formule (XIII) et celui de formule (XIV) est réalisée en présence d'au moins une base et à une température allant de 20°C à 150°C.

La base peut être choisie parmi les alcoolates alcalins, les carbonates alcalins, les carbonates alcalino-terreux, les hydroxydes alcalins, les hydroxydes alcalino-terreux et leurs mélanges.

Préférentiellement, la base est choisie parmi le méthanolate de sodium, le carbonate de potassium et la soude, plus préférentiellement le carbonate de potassium.

Préférentiellement, la quantité molaire de base est de 1,5 à 8 équivalents molaires, de préférence de 2 à 6 équivalents molaires par rapport à la quantité molaire de composé de formule (XIII).

Selon un mode de réalisation, il est possible d'ajouter un ou plusieurs catalyseurs choisi parmi un catalyseur de type sel d'argent (I), un catalyseur de transfert de phase de type ammonium quaternaire, et leur mélanges.

Les composés de formule (XIII) et (XIV) tels que définis ci-dessus sont disponibles commercialement auprès de fournisseurs tels que Sigma -Aldrich, Merk etc.

Selon un mode de réalisation préférentiel, le procédé de préparation d'un composé de formule (VI) comprend au moins les réactions successives suivantes : la réaction (c) suivit de la réaction (d) telles qu'elles ont été définies précédemment. Plus préférentiellement encore dans ce mode de réalisation, l'ajout de la quantité totale d'hydroxylamine est effectué en deux fois dans la réaction (c).

Selon un autre mode de réalisation préférentiel, le procédé de préparation d'un composé de formule (VI) comprend au moins les réactions successives suivantes : la réaction (a) suivi de la réaction (b) suivi de la réaction (c) puis suivi la réaction (d) telles qu'elles ont été définies précédemment. Plus préférentiellement de mode de réalisation préférentiel, l'ajout de la quantité totale d'hydroxylamine est effectué en deux fois dans la réaction (c).

Comme expliqué précédemment, les composés de formule (I), en particulier ceux de formule (VI), sont utilisés comme agent de fonctionnalisation. Ils peuvent être greffés sur un ou plusieurs polymères comprenant au moins une liaison carbone-carbone insaturée, en particulier ce polymère peut être un élastomère et plus particulièrement un élastomère diénique. Les composés de l'invention permettent avantageusement d'obtenir des polymères greffés (aussi appelés polymères modifiés), en particulier des élastomères diéniques greffés, quelle que soit la microstructure initiale du polymère, la seule condition étant que le polymère comprenne au moins une liaison carbone-carbone insaturée, de préférence une double liaison carbone-carbone.

Par élastomère (ou indistinctement caoutchouc) diénique, qu'il soit naturel ou synthétique, doit être compris de manière connue un élastomère constitué au moins en partie (i.e. un homopolymère ou un copolymère) d'unités monomères diènes (monomères porteurs de deux doubles liaisons carbone-carbone, conjuguées ou non).

Le greffage du polymère comprenant au moins une liaison carbone-carbone insaturée, se fait par réaction du polymère initial avec le composé de formule (I), en particulier le composé de formule (VI). Le greffage de ces composés est effectué par cycloaddition [3+2] du groupe Q desdits composés sur une liaison carbone-carbone insaturée de la chaîne du polymère. Le mécanisme de cette cycloaddition est notamment illustré dans le document WO2012/007441. Lors de cette réaction, ledit composé de formule (I), en particulier celui de formule (VI), forme des liaisons covalentes avec la chaîne du polymère.

Le greffage du composé de formule (I), en particulier du composé de formule (VI) peut être réalisée en masse, par exemple dans un mélangeur interne ou dans un mélangeur externe tel qu'un mélangeur à cylindres, ou en solution. Le procédé de greffage peut être effectué en solution en continu ou en discontinu. Le polymère modifié peut être séparé de sa solution par tout type de moyen connu par l'homme du métier et en particulier par une opération de stripping à la vapeur d'eau.

Les exemples qui suivent illustrent l'invention sans toutefois la limiter.

### EXEMPLES

### Détermination de la température de transition vitreuse

La température de transition vitreuse Tg des polymères sont mesures au moyen d'un calorimètre différentiel (« Differential Scanning Calorimeter ») selon la norme ASTM D3418-08.

### Caractérisations des molécules

L'analyse structurale ainsi que la détermination des puretés molaires des molécules de synthèse sont réalisées par une analyse RMN. Les spectres sont acquis sur un spectromètre Avance 3 400 MHz BRUKER équipé d'une sonde « large bande BBFO-zgrad 5 mm ». L'expérience RMN ¹H quantitative, utilise une séquence simple impulsion 30° et un délai de répétition de 3 secondes entre chacune des 64 acquisitions. Les échantillons sont solubilisés dans un solvant deutéré, le diméthylsulfoxide deutéré (DMSO) sauf indication contraire. Le solvant deutéré est également utilisé pour le signal de « lock ». Par exemple, la calibration est réalisée sur le signal des protons du DMSO deutéré à 2,44 ppm par rapport à une référence TMS à 0 ppm. Le spectre RMN ¹H couplé aux expériences 2D HSQC ¹H/¹³C et HMBC ¹H/¹³C permettent la détermination structurale des molécules (cf tableaux d'attribution). Les quantifications molaires sont réalisées à partir du spectre RMN 1D ¹H quantitatif.

### Molécules greffée sur élastomère diénique

La détermination du taux molaire du composé greffé testé sur un élastomère diénique est réalisée par une analyse RMN. Les spectres sont acquis sur un spectromètre 500 MHz BRUKER équipé d'une sonde « CryoSonde BBFO-zgrad-5 mm ». L'expérience RMN ¹H quantitative, utilise une séquence simple impulsion 30° et un délai de répétition de 5 secondes entre chaque acquisition. Les échantillons sont solubilisés dans un solvant deutéré, le chloroforme deutéré (CDCl₃) sauf indication contraire dans le but d'obtenir un signal de « lock ». Des expériences RMN 2D ont permis de vérifier la nature du motif greffé grâce aux déplacements chimiques des atomes de carbone et de proton.

### Mesure des masses molaires moyennes en nombre (Mn), en poids (Mw) et de l'indice de polydispersité des élastomères diéniques.

On utilise la chromatographie d'exclusion stérique ou SEC (Size Exclusion Chromatography). La SEC permet de séparer les macromolécules en solution suivant leur taille à travers des colonnes remplies d'un gel poreux. Les macromolécules sont séparées suivant leur volume hydrodynamique, les plus volumineuses étant éluées en premier.

Sans être une méthode absolue, la SEC permet d'appréhender la distribution des masses molaires d'un élastomère. À partir de produits étalons commerciaux, les différentes masses molaires moyennes en nombre (Mn) et en poids (Mw) peuvent être déterminées et l'indice de polymolécularité (Ip = Mw/Mn) calculé via un étalonnage dit de MOORE.

Il n'y a pas de traitement particulier de l'échantillon de l'élastomère avant analyse. Celui-ci est simplement solubilisé à une concentration d'environ 1 g/l, dans du chloroforme ou dans le mélange suivant : tétrahydrofurane + 1 % vol. de diisopropylamine + 1 % vol. de triéthylamine + 1 % vol. d'eau distillée (% vol. =% volume). Puis, la solution est filtrée sur filtre de porosité 0,45 µm avant injection.

L'appareillage utilisé est un chromatographe « WATERS alliance ». Le solvant d'élution est le mélange suivant : tétrahydrofurane + 1 % vol. de diisopropylamine + 1 % vol. de triéthylamine ou du chloroforme selon le solvant utilisé pour la mise en solution de l'élastomère. Le débit est de 0,7 ml/min, la température du système de 35°C et la durée d'analyse de 90 min. On utilise un jeu de quatre colonnes WATERS en série, de dénominations commerciales « STYRAGEL HMW7 », « STYRAGEL HMW6E » et deux « STYRAGEL HT6E ».

Le volume injecté de la solution de l'échantillon de l'élastomère est 100 µL. Le détecteur est un réfractomètre différentiel « WATERS 2410 » de longueur d'onde 810 nm. Le logiciel d'exploitation des données chromatographiques est le système «WATERS EM POWER».

Les masses molaires moyennes calculées sont relatives à une courbe d'étalonnage réalisée à partir de polystyrènes étalons commerciaux « PSS READY CAL-KIT ».

### Essai de traction :

Ces essais permettent de déterminer les contraintes d'élasticité et les propriétés à la rupture après cuisson. Sauf indication différente, ils sont effectués conformément à la norme française NF T 46-002 de septembre 1988. On mesure en première élongation (i.e., sans cycle d'accommodation) les modules sécants vrais (i.e., calculés en se ramenant à la section réelle de l'éprouvette), exprimés en MPa, à 100 % d'allongement (modules notés M100), à 300 % d'allongement (M300). Toutes ces mesures de traction sont effectuées dans les conditions normales de température et d'hygrométrie (23°C ± 2°C, 50 % ± 5 % d'humidité relative).

Les résultats sont indiqués en base 100 ; la valeur arbitraire 100 étant attribuée au témoin pour calculer et comparer ensuite M100 des différents échantillons testés. La valeur en base 100 pour l'échantillon à tester est calculée selon l'opération : (valeur de M100 de l'échantillon à tester/valeur de M100 du témoin) × 100. On effectue le même calcul pour M300 et pour le rapport M300/M100. On s'intéresse en particulier au rapport M300/M100, qui donne une indication des propriétés de renforcement. Plus la valeur du rapport M300/M100 est élevée, plus les propriétés de renforcement sont améliorées.

### Propriétés dynamiques

Les propriétés dynamiques ΔG^{∗} et tan(δ)max sont mesurées sur un viscoanalyseur (Metravib VA4000), selon la norme ASTM D5992-96. On enregistre la réponse d'un échantillon de composition vulcanisée (éprouvette cylindrique de 4 mm d'épaisseur et de de 400 mm² de section), soumis à une sollicitation sinusoïdale en cisaillement simple alterné, à la fréquence de 10Hz à 60°C. On effectue un balayage en amplitude de déformation de 0,1 % à 100 % (cycle aller) puis de 100 % à 0,1 % (cycle retour).

On effectue également ces mêmes mesures à une température de 100°C.

Les résultats exploités sont l'écart de module complexe de cisaillement dynamique entre les valeurs 0,1% et 100% de déformation à 60°C (ΔG^{∗}_{à 60°C retour} ; effet Payne) et le facteur de perte tan(δ).

Pour le cycle aller, on indique la valeur maximale de tan(δ) à 60°C noté tan(δ)_{max à 60°C aller}. Pour le cycle retour, on indique la valeur maximale de tan(δ) à 100°C noté tan(δ)_{max à 100°C retour}. Les résultats sont indiqués en base 100 ; la valeur arbitraire 100 étant attribuée au témoin pour calculer et comparer ensuite tan(δ)_{max à 60°C aller} des différents échantillons testés. La valeur en base 100 pour l'échantillon à tester est calculée selon l'opération : (valeur de tan(δ)_{max à} 60°C ₐₗₗₑᵣ de l'échantillon à tester/valeur de tan(δ)_{max à} 60°C ₐₗₗₑᵣ du témoin) × 100. De cette façon, un résultat inférieur à 100 indique une diminution de l'hystérèse (donc une amélioration des propriétés hystérétiques) qui correspond à une amélioration de la performance de résistance au roulement. Le même calcul est effectué pour les valeurs de tan(δ)ₘₐₓ à _{100°C retour} et (ΔG^{∗}_{à} 60°C ᵣₑₜₒᵤᵣ) afin d'exprimer les résultats en base 100.

Un résultat inférieur à 100 pour ΔG^{∗}_{à 60°C retour} indique une meilleure dispersion de la charge renforçante dans la composition de caoutchouc.

### I-Synthèse des composés D et I

### I-AI Synthèse du composé D : Oxyde de 2-((2-oxo-1,3-dioxolan-4-yl)methoxy)-1-naphthonitrile

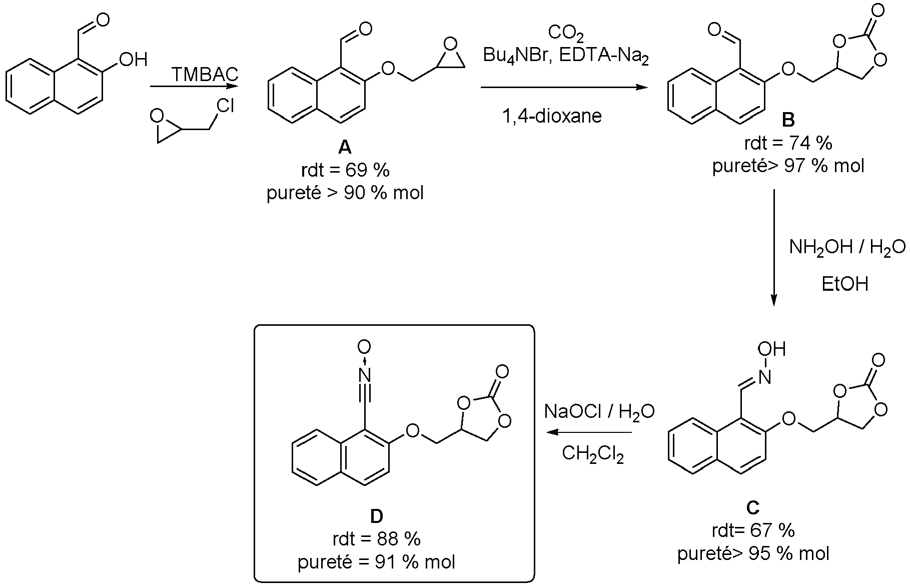

L'oxyde de 2-((2-oxo-1,3-dioxolan-4-yl)méthoxy)-1-naphthonitrile est synthétisé en 4 étapes qui sont décrites ci-après. Tous les composés chimiques utilisés lors de cette synthèse proviennent de « Sigma Aldrich ».

### Étape 1 : préparation du 2-(oxiran-2-ylméthoxy)-1-naphthaldéhyde (composé A)

Une solution de 2-hydroxy-1-naphtaldéhyde (35,0 g ; 0,203 mol,) dans l'épichlorohydrine (270 ml ; 320,0 g ; 3,456 mol ; 17 Eq.) est chauffée pendant 3-5 minutes à une température de 130°C, puis, du chlorure de triméthylbenzylammonium (TMBAC ; 3,8 g ; 0,020 mol ; 0,1 Eq.) est ajouté. Le milieu réactionnel est chauffé jusqu'à ébullition (température du bain = 130-134°C) et agité à cette température pendant 15 minutes. Après cette période, la solution est refroidie à 30-40°C, puis 400 ml de chloroforme sont ajoutés. La solution organique est lavée 4 fois par 150 ml d'eau et la phase organique est séparée puis concentrée sous pression réduite (11 mbar, température du bain = 50°C) pour conduire à 76,58 g d'une huile. Ce résidu huileux est repris par 90 ml de 2-propanol et le mélange est agité pendant 5 à 10 min. La suspension obtenue est ensuite placée pendant 4-5 heures à -18°C. Le précipité obtenu est alors filtré et lavé sur le filtre par du 2-propanol froid (T = - 18°C) (3 fois 20 ml). Le produit est séché à température ambiante et sous pression atmosphérique.

Un solide blanc de point de fusion 94,0-97,5°C est obtenu avec un rendement de 69 % (32,13 g ; 0,141 mol). La pureté molaire est supérieure à 90 % (RMN ¹H).

Le 2-hydroxy-1-naphtaldéhyde est commercial. Il peut par exemple être obtenu chez « Sigma Aldrich » (CAS 708-06-5).

### Étape 2 : Synthèse du 2-((2-oxo-1,3-dioxolan-4-yl)méthoxy)-1-naphthaldéhyde (composé B)

Le composé A, 2-(oxiran-2-ylméthoxy)-1-naphthaldéhyde (16,5 g ; 72,3 mmol), est mélangé avec du bromure de tétrabutylammonium (1,17 g; 3,61 mmol; 0,05 Eq.) et de l'acide d'éthylènediaminetétraacétique dihydrate sodique (1,17 g ; 3,14 mmol ; 0,04 Eq.) dans 500 ml de 1,4-dioxane. Ce mélange est chauffé à 100°C (température bain) sous atmosphère de CO₂ pendant 14-16 heures. Du CO₂ est additionné périodiquement par barbotage du milieu pour garder la pression de CO₂ constante. Après retour du mélange à température ambiante, le précipité est filtré et lavé sur filtre par du 1,4-dioxane (2 fois 10 ml). Le filtrat est concentré sous pression réduite (75 mbar, température bain 45°C) jusqu'à obtenir un résidu visqueux (41,23 g). De l'acétate d'éthyle (20 ml) et de l'éther de pétrole (30 ml) sont ajoutés (fraction volumique 40/60). Après 10-15 min d'agitation à température ambiante, le précipité obtenu est filtré et lavé sur filtre par un mélange d'acétate d'éthyle/éther de pétrole (2 fois par un mélange d'acétate d'éthyle/éther de pétrole : 5 ml/10 ml), puis par de l'eau (3 fois 10 ml) et enfin par de l'éther de pétrole (20 ml). Un solide blanc (16,85 g) est obtenu avec un rendement de 86 %.

Ce solide est ensuite dissous dans de l'alcool éthylique (100 ml). Après 10 min agitation à température d'ébullition puis retour à température ambiante (23°C), le milieu réactionnel est refroidi jusqu'à +4°C et conservé à cette température pendant 15-20 heures. Le précipité est filtré et lavé sur filtre par de l'éthanol (2 fois 10 ml) puis séché sous air à température ambiante. Le produit désiré (poudre blanche de point de fusion 158-159°C) est obtenu avec un rendement de 74 % (14,52 g ; 53,33 mmol) et une pureté molaire supérieure à 97 %.

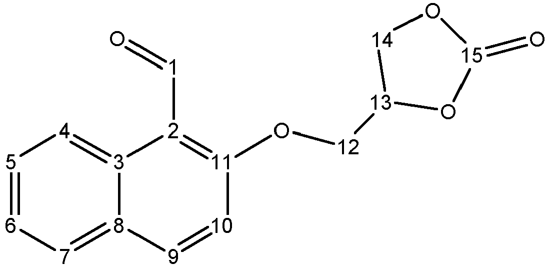

**[Table 1]**

| **N°** | **δ ¹H(ppm)** | **δ ¹³C(ppm)** |
|---|---|---|
| 1 | 10,68 | 190,8 |
| 2 | / | 116,0 |
| 3 | / | 130,5 |
| 4 | 9,04 | 123,9 |
| 5 | 7,60 | 129,8 |
| 6 | 7,43 | 124,9 |
| 7 | 7,90 | 128,5 |
| 8 | / | 128,4 |
| 9 | 8,25 | 137,9 |
| 10 | 7,52 | 114,5 |
| 11 | / | 162,6 |
| 12 | 4,49-4,59 | 69,1 |
| 13 | 5,23 | 74,6 |
| 14 | 4,50-4,63 | 66,1 |
| 15 | / | 154,7 |

| | | |
|---|---|---|
| Solvant : DMSO | | |

### Étape 3 : Synthèse de l'oxime 2-((2-oxo-1,3-dioxolan-4-yl)méthoxy)-1-naphthaldéhyde (composé C)

À une solution du composé B, 2-((2-oxo-1,3-dioxolan-4-yl)méthoxy)-1-naphthaldéhyde (5,00 g ; 18,37 mmol), dans l'éthanol (50 ml) à 35°C (température bain) est ajoutée une solution d'hydroxylamine (50 % en solution dans l'eau ; 1,82 g ; 27,5 mmol ; 1,5 Eq.) dans l'éthanol (5 ml). Le milieu réactionnel est chauffé jusqu'à 40°C puis agité à cette température pendant 8 heures. Un deuxième ajout de solution d'hydroxylamine (50 % en solution dans l'eau ; 0,61 g ; 9,2 mmol ; 0,5 Eq.) dans l'éthanol (25 ml) est réalisé. Le milieu réactionnel est agité à 40°C pendant 7 heures. Après de refroidissement à température ambiante, le milieu réactionnel est dilué par addition d'eau à 0°C (450 ml) sur une période de 15-20 minutes. Après 10 minutes d'agitation, le précipité est filtré et lavé sur filtre par de l'eau (2 fois 10 ml).

Un solide blanc de point de fusion 182-183°C est obtenu avec un rendement de 67 % (3,52 g ; 12,25 mmol) et de pureté molaire supérieure à 95 %.

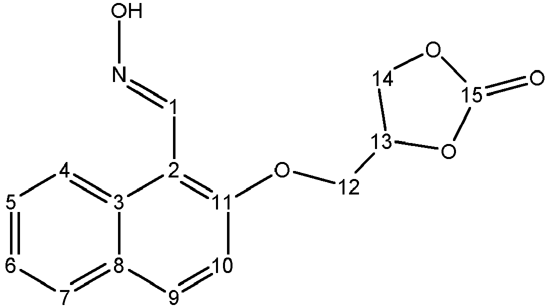

**[Table 2]**

| **N°** | **δ ¹H(ppm)** | **δ ¹³C(ppm)** |
|---|---|---|
| 1 | 8,60 | 144,8 |
| 2 | / | 114,2 |
| 3 | / | 130,7 |
| 4 | 8,82 | 125,7 |
| 5 | 7,49 | 127,6 |
| 6 | 7,37 | 124, |
| 7 | 7,85 | 128,3 |
| 8 | / | 129,1 |
| 9 | 7,95 | 131,5 |
| 10 | 7,42 | 114,6 |
| 11 | / | 154,9 |
| 12 | 4,36-4,46 | 69,0 |
| 13 | 5,17 | 74,8 |
| 14 | 4,41-4,62 | 65,9 |
| 15 | / | 154,7 |

| | | |
|---|---|---|
| Solvant : DMSO | | |

### Étape 4 : Synthèse de l'oxyde de 2-((2-oxo-1,3-dioxolan-4-yl)méthoxy)-1-naphthonitrile (composé D)

À une suspension d'oxime 2-((2-oxo-1,3-dioxolan-4-yl)méthoxy)-1-naphthaldéhyde (produit C) (3,42 g; 11,91 mmol) dans le dichlorométhane (75 ml) à une température de +1°C est ajoutée au goutte à goutte une solution aqueuse de NaOCl dans l'eau (21,5 ml ; 19,05 mmol ; 1,6 Eq. ; solution en chlore actif > 4 %) pendant 3-5 minutes. Le milieu réactionnel est agité pendant 70-80 minutes à cette température. Le précipité est filtré et lavé sur filtre par du CH₂Cl₂ (10 ml), puis par de l'eau (2 fois 15 ml) et enfin par un mélange de dichlorométhane/éther de pétrole (fraction volumique 50/50) (10 ml/10 ml) Après séchage sous pression atmosphérique et à température ambiante, un solide blanc de point de fusion 157-158°C est obtenu avec un rendement de 88 % (2,976 g ; 10,43 mmol) et de pureté supérieure à 91 % molaire.

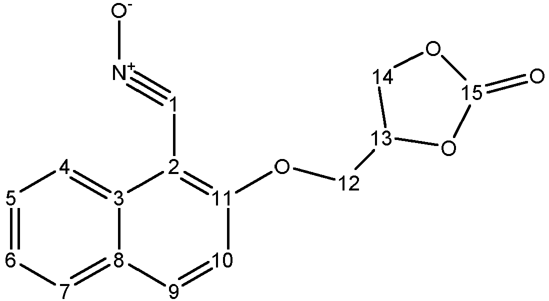

**[Table 3]**

| **N°** | **δ ¹H(ppm)** | **δ ¹³C(ppm)** |
|---|---|---|
| 1 | / | / |
| 2 | / | 95,6 |
| 3 | / | 133,2 |
| 4 | 7,87 | 123,3 |
| 5 | 7,64 | 129,1 |
| 6 | 7,47 | 125,2 |
| 7 | 7,96 | 128,7 |
| 8 | / | 128,3 |
| 9 | 8,14 | 133,3 |
| 10 | 7,53 | 114,2 |
| 11 | / | 159,9 |
| 12 | 4,49-4,60 | 68,9 |
| 13 | 5,19 | 74,6 |
| 14 | 4,41-4,62 | 65,8 |
| 15 | / | 154,6 |

| | | |
|---|---|---|
| Solvant DMSO | | |

### I-b/ Synthèse du composé I: oxyde de 2,4,6-triméthyl-3-((2-oxo-1,3-dioxolan-4-yl)méthoxy)benzonitrile

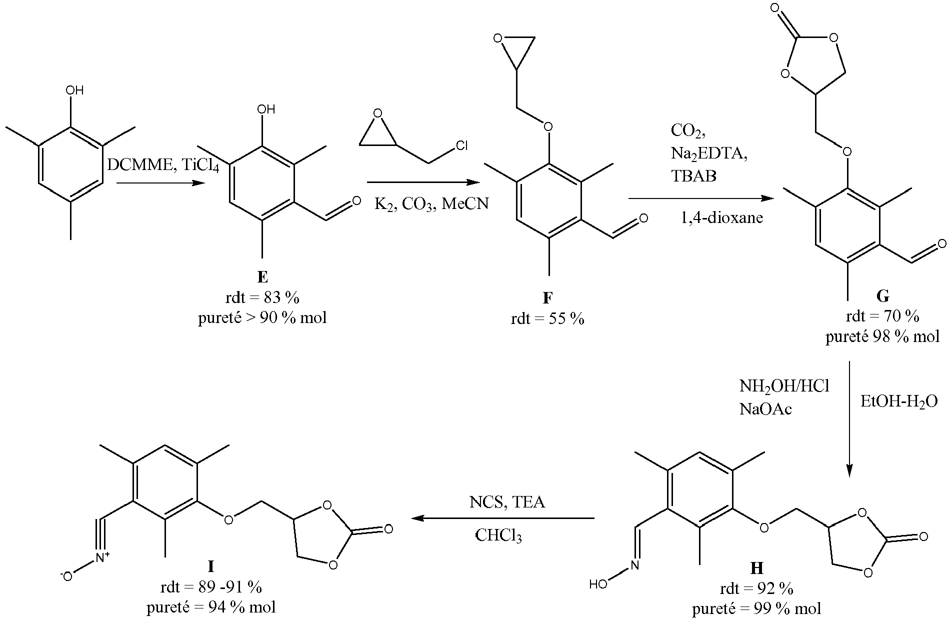

L'oxyde de 2,4,6-triméthyl-3-((2-oxo-1,3-dioxolan-4-yl)méthoxy)benzonitrile est synthétisé en 5 étapes décrites ci-après. Tous les composés chimiques utilisés lors de cette synthèse proviennent de chez « Sigma Aldrich ».

### Etape 1 : Préparation du 3-hydroxy-2,4,6-triméthylbenzaldéhyde (composé E)

Ce composé peut être obtenu à partir du mésitol et du dichlorométhyl méthyl éther (DCMME) selon une procédure décrite dans l'article suivante: Yakubov, A.P.; Tsyganov, D.V.; Belen'kii, L.I.; Krayushkin, M.M. Bulletin of the Academy of Sciences of the USSR, Division of the Chemical Science (English Translation); vol. 40; nb. 7.2; (1991); p. 1427-1432; Izvestiya Akademii Nauk SSSR, Seriya Khimicheskaya; nb. 7; (1991); p. 1609-1615.

Le composé E ayant un point de fusion de 108-109°C est obtenu avec un rendement de 83 % et une pureté molaire supérieure à 90 % (RMN ¹H).

Le mésitol est commercial. Il peut être obtenu par exemple chez « Sigma-Aldrich » (CAS 527-60-6].

### Etape 2 : Préparation du 2,4,6-triméthyl-3-(oxiran-2-ylméthoxy)benzaldéhyde (composé F)

Au mélange de composé E, le 3-hydroxy-2,4,6-triméthylbenzaldéhyde (30,00 g; 0,183 mol), et d'épichlorhydrine (42,3 g; 0,457 mol) dans l'acétonitrile (80 ml) est ajouté du potassium carbonate (37,9 g; 0,274 mol). Le milieu réactionnel est chauffé pendant 3 heures à la température de 60°C et ensuite pendant 2,5-3 heures à la température de 70°C. Après un refroidissement à une température de 40-50°C, le mélange réactionnel est dilué par un mélange d'eau (250 ml) et d'acétate d'éthyle (250 ml) et est agité pendant 10 minutes. La phase organique est séparée et lavée par d'eau (4 fois 100 ml). Le solvant est évaporé sous pression réduite (température bain = 40°C ; 12 mbar). Une huile jaune (39,116 g) est obtenue.

Après séparation par chromatographie sur colonne (SiO₂; acétate d'éthyle (AE) : éther de pétrole (PE) = 1:4) et récupération des fractions du produit d'intérêt ; les solvants sont évaporés sous pression réduite (température bain = 40°C ; 11 mbar). L'éther de pétrole (150 ml) est ajouté au résidu obtenu après évaporation et ce mélange est placé à -18°C pendant 2 heures. Le précipité obtenu est filtré, lavé par l'éther de pétrole (3 fois 25 ml) et enfin séché à l'air.

Un solide blanc (21,916g) est obtenu avec un rendement de 55 %.

### Étape 3 : Préparation du 2,4,6-triméthyl-3-((2-oxo-1,3-dioxolan-4-yl)méthoxy) benzaldéhyde (Composé G)

Le composé E, le 2,4,6-triméthyl-3-(oxiran-2-ylméthoxy)benzaldéhyde (5,00 g; 22,70 mmol) est mélangé avec du bromure de tétra-n-butylammonium (TBAB ; 0,366 g; 1,135 mmol) et Na₂EDTA dihydrate (0,422 g; 1,135 mmol) dans 100 ml de 1,4-dioxane à une température bain égale à 110°C sous atmosphère de CO₂. Du CO₂ est additionné périodiquement par barbotage du milieu pendant 7-8 heures pour garder la pression de CO₂ constante. La pression interne est maintenue par un ballon. Une conversion de 60-65% est atteinte au bout de 14 heures. Après refroidissement à une température de 60°C, le précipité est filtré et lavé par 1,4-dioxane (2 fois 5 ml). Le filtrat est concentré sous pression réduite (température bain = 50°C ; 30 mbar) pour conduire à 5,323 g d'une huile brune.

Après séparation par chromatographie sur colonne (SiO₂; acétate d'éthyle/éther de pétrole = 1:1) et récupération des fractions du produit d'intérêt, les solvants sont évaporés sous pression réduite (température bain = 40°C ; 20 mbar). L'éther de pétrole (5 ml) est ajouté pour provoquer une précipitation rapide. Le précipité est filtré, lavé par l'éther de pétrole (2 fois 5 ml) et enfin séché à l'air.

Un solide blanc (1,835 g) est obtenu avec un rendement de 31 %. La pureté molaire est supérieure à 98% (RMN ¹H).

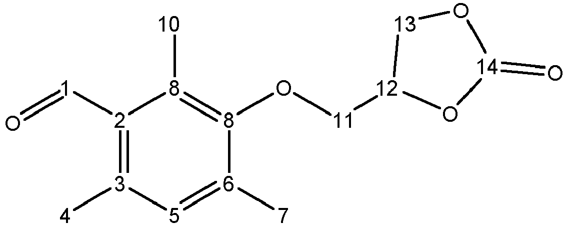

**[Table 4]**

| **N°** | **δ ¹H (ppm)** | **δ ¹³C (ppm)** |
|---|---|---|
| 1 | 10,38 | 193,7 |
| 2 | / | 131,5 |
| 3 | / | 136,3 |
| 4 | 2,41 | 19,3 |
| 5 | 6,96 | 131,7 |
| 6 | / | 136,3 |
| 7 | 2,20 | 16,1 |
| 8 | / | 152,7 |
| 9 | / | 133,1 |
| 10 | 2,39 | 11,7 |
| 11 | 3,92 | 71,3 |
| 12 | 5,07 | 75,3 |
| 13 | 4,41-4,60 | 65,8 |
| 14 | / | 154,8 |

| | | |
|---|---|---|
| Solvant DMSO | | |

### Étape 4 : Préparation de l'oxime 2,4,6-triméthyl-3-((2-oxo-1,3-dioxolan-4-yl)méthoxy) benzaldéhyde (composé H)

À une suspension de composé G, le 2,4,6-triméthyl-3-((2-oxo-1,3-dioxolan-4-yl)méthoxy) benzaldéhyde (1,200g; 4,54 mmol), dans de l'éthanol (50 ml), on ajoute, à température ambiante, une solution d'acétate de sodium (0,559 g; 6,81 mmol) et d'hydrochlorure d'hydroxylamine (0,473 g; 6,81 mmol) dans d'eau (50 ml). Le mélange réactionnel est agité pendant 3 heures à température ambiante. Ensuite, on ajoute un volume d'eau à 0°C (50 ml) et on laisse agiter pendant 15 minutes supplémentaires. Le précipité obtenu est filtré, lavé par d'eau (3 fois 30 ml) et séché à l'air.

Un solide blanc (1,161 g) ayant un point de fusion de 144-145°C est obtenu avec un rendement de 92 %. La pureté molaire est supérieure à 98 % (RMN ¹H).

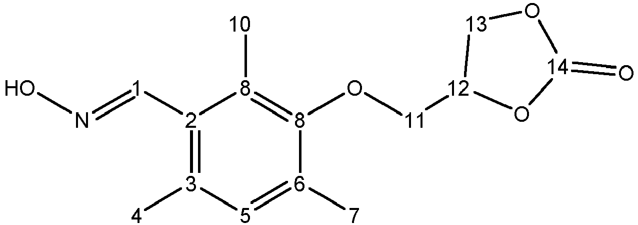

**[Table 5]**

| **N°** | **δ ¹H(ppm)** | **δ ¹³C(ppm)** |
|---|---|---|
| 1 | 8,31 | 147,4 |
| 2 | / | 129,4 |
| 3 | / | 132,4 |
| 4 | 2,19 | 20,12 |
| 5 | 6,87 | 130,5 |
| 6 | / | 130,4 |
| 7 | 2,15 | 15,7 |
| 8 | / | 152,4 |
| 9 | / | 129,4 |
| 10 | 2,18 | 13,0 |
| 11 | 3,89 | 71,1 |
| 12 | 5,05 | 75,4 |
| 13 | 4,40-4,59 | 65,8 |
| 14 | / | 154,8 |

| | | |
|---|---|---|
| Solvant DMSO | | |

### Etape 5 : synthèse de l'oxyde 2,4,6-trimethyl-3-((2-oxo-1,3-dioxolan-4-yl)methoxy)benzonitrile (composé I)

À une suspension du composé H, l'oxime 2,4,6-triméthyl-3-((2-oxo-1,3-dioxolan-4-yl)méthoxy) benzaldéhyde (1,01 g; 3,62 mmol) dans CHCl₃ (50 ml) refroidie jusqu'à 0-2°C, on ajoute de TEA (0,476 g; 4,70 mmol) en une fois et du N-chlorosucinimide (NCS , 0,531 g; 3,98 mmol) par portions pendant 1-2 minutes. Le mélange réactionnel est agité entre 0-3°C pendant 1 heure. Puis la phase organique est lavée par de l'eau (4 fois 100 ml) et concentrée sous pression réduite (température bain = 25°C ; 10 mbar) pour obtenir d'une huile jaune (1,606 g). Ensuite on ajoute du méthyl tert-bytul éther (MTBE, 5 ml). Le précipité obtenu est filtré, lavé par MTBE : éther de pétrole = 1:1 (2 fois 5 ml) et séché à l'air.

Un solide blanc (0,912 g) de point de fusion 128-129°C est obtenu avec un rendement de 91 %. La pureté molaire est supérieure à 94 % (RMN ¹H).

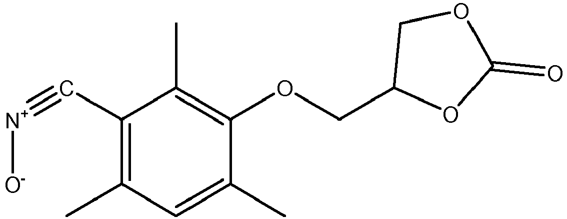

**[Table 6]**

| **N°** | **δ ¹H(ppm)** | **δ ¹³C(ppm)** |
|---|---|---|
| 1 | / | / |
| 2 | / | 112,3 |
| 3 | / | 137,3 |
| 4 | 2,28 | 19,7 |
| 5 | 7,03 | 130,3 |
| 6 | / | 134,4 |
| 7 | 2,18 | 15,9 |
| 8 | / | 152,3 |
| 9 | / | 134,1 |
| 10 | 2,27 | 14,3 |
| 11 | 3,94 | 65,7 |
| 12 | 5,06 | 75,2 |
| 13 | 4,39-4,58 | 71,3 |
| 14 | / | 154,8 |

| | | |
|---|---|---|
| Solvant : DMSO | | |

### II Greffage de polymères avec les composés D et I

### II-a/ Fabrication d'un copolymère styrène-butadiène (SBR) greffé par l'oxyde de 2-((2-oxo-1,3-dioxolan-4-yl)méthoxy)-1-naphthonitrile (composé D)

On incorpore de l'oxyde de 2-((2-oxo-1,3-dioxolan-4-yl)méthoxy)-1-naphthonitrile (composé D) (0,35g; 1,21 mmol) à 15 g de SBR (contenant 26,5% en poids de styrène par rapport au poids total du copolymère et 24% en poids d'unité butadiène-1,2, par rapport au poids de la partie butadiènique, 28 % d'unité butadiène-1,4 cis, par rapport au poids de la partie butadiènique et 48 % d'unité butadiène-1,4 trans, par rapport au poids de la partie butadiènique, de Mn=120000 g/mol et Ip=1,22) sur outil à cylindres (mélangeur externe à 23°C). Le mélange est homogénéisé en 15 passes portefeuille. Cette phase de mélangeage est suivie d'un traitement thermique à 120°C pendant 10 minutes sous presse à 10 bars de pression.

Les résultats du greffage d'après l'analyse RMN ¹H sont présentés dans le tableau ci-après.

### II-b/ Fabrication d'un polyisoprène de synthèse (IR) greffé par l'oxyde de 2-((2-oxo-1,3-dioxolan-4-yl)méthoxy)-1-naphthonitrile (composé D)

On incorpore de l'oxyde de 2-((2-oxo-1,3-dioxolan-4-yl)méthoxy)-1-naphthonitrile (0,32 g ; 1,1 mmol) à 15 g de polyisoprène synthétique (contenant 99,35% en poids d'unité isoprène-1,4 cis et 0,65% d'unité isoprène 3,4 et de Mn=550000 g/mol et Ip=2,4) sur outil à cylindres (mélangeur externe à 23°C). Le mélange est homogénéisé en 15 passes portefeuille. Cette phase de mélangeage est suivie d'un traitement thermique à 120°C pendant 10 minutes sous presse à 10 bars de pression.

Les résultats du greffage d'après l'analyse RMN ¹H sont présentés dans le tableau ci-après.

### II-c/ Fabrication d'un caoutchouc naturel (NR) greffé par l'oxyde de 2-((2-oxo-1,3-dioxolan-4-yl)méthoxy)-1-naphthonitrile (composé D)

On incorpore de l'oxyde de 2-((2-oxo-1,3-dioxolan-4-yl)méthoxy)-1-naphthonitrile (0,32 g ; 1,1 mmol) à 15 g d'un caoutchouc naturel sur outil à cylindres (mélangeur externe à 23°C). Le mélange est homogénéisé en 15 passes portefeuille. Cette phase de mélangeage est suivie d'un traitement thermique à 120°C pendant 10 minutes sous presse à 10 bars de pression.

Les résultats du greffage d'après l'analyse RMN ¹H sont présentés dans le tableau ci-après.

### II-d/ Fabrication d'un copolymère éthylène-butadiène (EBR) greffé par l'oxyde de 2-((2-oxo-1,3-dioxolan-4-yl)méthoxy)-1-naphthonitrile (composé D)

On incorpore de l'oxyde de 2-((2-oxo-1,3-dioxolan-4-yl)méthoxy)-1-naphthonitrile (0,451g ; 1,44 mmol) à 15g d'EBR (contenant 80 % en mole d'éthylène et 20 % en mole de butadiène, Mn= 187000 g/mol, Ip =1,36) sur outil à cylindres (mélangeur externe à 23°C). Le mélange est homogénéisé en 15 passes portefeuille. Cette phase de mélangeage est suivie d'un traitement thermique à 120°C pendant 10 minutes sous presse à 10 bars de pression.

Les résultats du greffage d'après l'analyse RMN ¹H sont présentés dans le tableau ci-après.

**[Table 7]**

| Élastomère | Taux de greffage visé (% molaire) | Taux de composé D greffé (% molaire) | Rendement de greffage (en %) |
|---|---|---|---|
| SBR | 0,50 | 0,40 | 80 |
| IR | 0,50 | 0,14 | 28 |
| NR | 0,50 | 0,10 | 20 |
| EBR | 0,35 | 0,14 | 40 |

### II-e/-Fabrication d'un polyisoprène de synthèse (IR) greffé par l'oxyde 2,4,6-trimethyl-3-((2-oxo-1,3-dioxolan-4-yl)methoxy)benzonitrile (composé I)

On incorpore l'oxyde 2,4,6-trimethyl-3-((2-oxo-1,3-dioxolan-4-yl)methoxy)benzonitrile (0,3 g ; 1,1 mmol) à 15 g de polyisoprène synthétique (contenant 99,35% en poids d'unité isoprène-1,4 cis et 0,65% d'unité isoprène 3,4 et de Mn=375000 g/mol et Ip=3,6) sur outil à cylindres (mélangeur externe à 23°C). Le mélange est homogénéisé en 15 passes portefeuille. Cette phase de mélangeage est suivie d'un traitement thermique à 120°C pendant 10 minutes sous presse à 10 bars de pression.

Les résultats du greffage d'après l'analyse RMN ¹H sont présentés dans le tableau ci-après.

### II-f/Fabrication d'un copolymère styrène-butadiène (SBR) greffé par l'oxyde 2,4,6-trimethyl-3-((2-oxo-1,3-dioxolan-4-yl)methoxy)benzonitrile (composé I)

On incorpore l'oxyde 2,4,6-trimethyl-3-((2-oxo-1,3-dioxolan-4-yl)methoxy)benzonitrile (composé I) (0,34 g; 1,21 mmol) à 15 g de SBR (contenant 26,5% en poids de styrène par rapport au poids total du copolymère et 24% en poids d'unité butadiène-1,2, par rapport au poids de la partie butadiènique, 28 % d'unité butadiène-1,4 cis, par rapport au poids de la partie butadiènique et 48 % d'unité butadiène-1,4 trans, par rapport au poids de la partie butadiènique, de Mn=120000 g/mol et Ip=1,22) sur outil à cylindres (mélangeur externe à 23°C). Le mélange est homogénéisé en 15 passes portefeuille. Cette phase de mélangeage est suivie d'un traitement thermique à 120°C pendant 10 minutes sous presse à 10 bars de pression.

Les résultats du greffage d'après l'analyse RMN ¹H sont présentés dans le tableau ci-après.

**[Table 8]**

| Élastomère | Taux de greffage visé (% molaire) | Taux de composé I greffé (% molaire) | Rendement de greffage (en %) |
|---|---|---|---|
| SBR | 0,50 | 0,50 | 100 |
| IR | 0,50 | 0,18 | 36 |

### III- Composition de caoutchouc

### III-1/ préparation des compositions de caoutchouc

Cet essai a pour but de démontrer les performances améliorées de compositions de caoutchouc comprenant un polymère greffé avec le composé dipolaire azoté de l'invention comparée à une composition de caoutchouc comprenant un polymère non greffé et comparée à une composition de caoutchouc comprenant un polymère avec un composé dipolaire azoté de l'art antérieur.

On prépare ainsi trois compositions selon le procédé décrit ci-dessous, à base d'un élastomère SBR, renforcées majoritairement par de la silice ; ces compositions se distinguent les unes des autres par la présence ou non d'un agent de fonctionnalisation et par la nature de cet agent de fonctionnalisation, comme suit :
- La composition témoin T1, non conforme à l'invention comprenant un élastomère A qui est un SBR non greffé contenant 26,5 % en poids de styrène par au poids total de l'élastomère, et 24% en poids d'unités butadiène 1,2 par rapport au poids de la partie butadiènique ; de Mn= 120 000 g/mol et d'indice de polydispersité Ip=1,22) ;
- La composition C1, non conforme à l'invention comprenant un élastomère B qui est un SBR greffé par l'oxyde de 2,4,6-trimethyl-3-[2-(2-oxo1-imidazolidinyl)éthoxy]-benzonitrile ; le SBR contenant 26,5 % en poids de styrène par au poids total de l'élastomère, et 24% en poids d'unités butadiène 1,2 par rapport au poids de la partie butadiènique ; de Mn= 120 000 g/mol et d'indice de polydispersité Ip=1,22) ;
- La composition C2, conforme à l'invention, comprenant l'élastomère C qui est un SBR greffé par l'oxyde de 2-((2-oxo-1,3-dioxolan-4yl)méthoxy)-1-naphthonitrile ; le SBR contenant 26,5 % en poids de styrène par au poids total de l'élastomère, et 24% en poids d'unités butadiène 1,2 par rapport au poids de la partie butadiènique ; de Mn= 120 000 g/mol et d'indice de polydispersité Ip=1,22).

### Obtention de l'élastomère B greffé

On incorpore de l'oxyde de 2, 4, 6-triméthyl-3-[2-(2-oxo-1-imidazolidinyl)éthoxy]-benzonitrile (7.797 g ; 28.3 mmol ; 90 % molaire de pureté) à 50 g de SBR (élastomère A) sur outil à cylindres (mélangeur externe à 23°C). Le mélange est homogénéisé en 15 passes portefeuille. Cette phase de mélangeage est suivie d'un traitement thermique à 120°C pendant 10 minutes sous presse à 10 bars de pression.

L'analyse par RMN ¹H a permis de démontrer un taux molaire de greffage de l'oxyde de 2, 4, 6-triméthyl-3-[2-(2-oxo-1-imidazolidinyl)éthoxy]-benzonitrile qui est égal à 3,14 % et un rendement molaire de greffage qui est égal à 100 %.

L'oxyde de 2, 4, 6-triméthyl-3-[2-(2-oxo-1-imidazolidinl-yl)éthoxy]-benzonitrile est synthétisé selon le protocole décrit l'exemple 1 du document WO2012/007684.

### Obtention de l'élastomère C greffé

On incorpore de l'oxyde de 2-((2-oxo-1,3-dioxolan-4-yl)méthoxy)-1-naphthonitrile (6,908 g ; 24,2 mmol ; 92 % molaire de pureté) à 50 g de SBR (élastomère A) sur outil à cylindres (mélangeur externe à 23°C). Le mélange est homogénéisé en 15 passes portefeuille. Cette phase de mélangeage est suivie d'un traitement thermique à 120°C pendant 10 minutes sous presse à 10 bars de pression.

L'analyse par RMN ¹H a permis de mesurer un taux molaire de greffage de l'oxyde de 2-((2-oxo-1,3-dioxolan-4-yl)méthoxy)-1-naphthonitrile qui est égal à 2,6 % et un rendement molaire de greffage qui est égal à 94 %.

L'oxyde de 2-((2-oxo-1,3-dioxolan-4-yl)méthoxy)-1-naphthonitrile est le composé D dont le protocole de synthèse a été décrit ci-dessus.

### Préparation des compositions de caoutchouc

On introduit dans un mélangeur interne Polylab de 85 cm³ dont le taux de remplissage final environ 70% en volume et dont la température initiale de cuve est d'environ 100°C, successivement l'élastomère greffé ou non greffé, la charge renforçante et les autres additifs à l'exception du système de vulcanisation. On conduit alors un travail thermomécanique (phase non-productive) en une étape (durée totale du malaxage égale à environ 5 min), jusqu'à atteindre une température maximale de « tombée » allant de 145 à 165°C. On récupère le mélange ainsi obtenu, on le refroidit puis on ajoute le système de vulcanisation sur un mélangeur externe pour y réaliser une seconde phase de travail mécanique à environ 80°C pendant environ 5 à 6 min.

Les compositions ainsi obtenus sont ensuite calandrées soit sous la forme de plaques d'épaisseur de 2 à 3 mm ou de feuilles fines de caoutchouc pour la mesure de leurs propriétés physiques ou mécaniques

La formulation des compositions de caoutchouc est donnée dans le tableau suivant et leurs propriétés après cuisson (environ 60 min à 150°C) sont présentées dans le tableau ci-après. Les quantités sont exprimées en parties pour 100 parties en poids d'élastomère (pce).

**[Table 9]**

| **Composition** | **T1** | **C1** | **C2** |
|---|---|---|---|
| Élastomère A | 100 | (-) | (-) |
| Élastomère B | (-) | 100 | (-) |
| Élastomère C | (-) | (-) | 100 |
| Noir de carbone(1) | 1 | 1 | 1 |
| Silice (2) | 67 | 67 | 67 |
| Résine plastifiante(3) | 31 | 31 | 31 |
| Antioxydant (4) | 3 | 3 | 3 |
| Paraffine | 1 | 1 | 1 |
| Agent de recouvrement (5) | 5,36 | 5,36 | 5,36 |
| Diphénylguanidine (6) | 2,5 | 2,5 | 2,5 |
| Acide stéarique (7) | 3 | 3 | 3 |
| ZnO (8) | 0,9 | 0,9 | 0,9 |
| Soufre | 2,3 | 2,3 | 2,3 |
| CBS (9) | 1 | 1 | 1 |

(1) Noir de carbone de grade ASTM N234 commercialisé par Cabot ;
(2) Silice « Zeosil 1165 MP » de la société Solvay de surface spécifique BET est de 160 ₘ²/g ;
(3) Résine dicyclopentadiène/C9 hydrogénée « E5600 BR » commercialisée par Exxon Mobil ;
(4) 1,3-diméthylbutyl-N-phényl-para-phénylènediamine (« Santoflex 6-PPD » commercialisée par la société Flexsys ;
(5) Triméthoxy(octyl)silane commercialisé par Sigma Aldrich ;
(6) Diphénylguanidine (« Perkacit » DPG de la société Flexsys) ;
(7) Stéarine (« Pristerene 4931 » - société Uniqema) ;
(8) oxyde de zinc (grade industriel - société Umicore) ;
(9) N-cyclohexyl-2-benzothiazyl-sulfénamide (« Santocure CBS » société Flexys).

**[Table 10]**

| | T1 | C1 | C2 |
|---|---|---|---|
| ΔG^{∗} _{à 60°C retour} | 100 | 12 | 18 |
| Tan(δ)_{max à 60°C aller} | 100 | 91 | 68 |
| Tan(δ)_{max à 100°C retour} | 100 | 78 | 44 |
| M100 à 23°C | 100 | 227 | 277 |
| M300 à 23°C | 100 | 239 | 613 |
| M300/M100 à 23°C | 100 | 105 | 221 |

Au vu du tableau ci-dessus, on constate, comme attendu, que la composition C1 non conforme à l'invention qui comprend l'élastomère greffé avec l'oxyde de 2, 4, 6-trimethyl-3-[2-(2-oxo-1-imidazolidinl-yl)éthoxy]-benzonitrile présente une diminution de l'hystérèse (Tan(δ)_{max à 60°C aller} et de Tan(δ)_{max à 1000°C retour} ) par rapport à la composition T1 témoin, donc des propriétés hystérétiques améliorées par rapport à la composition T1 témoin qui ne comprend pas d'élastomère greffé. La composition C1 non conforme présente également des propriétés de renforcement (augmentation du rapport M300/M100) améliorées par rapport à la composition T1 témoin.

La valeur de ΔG^{∗}_{à 60°C retour} de la composition C2 conforme à l'invention est significativement inférieure à celle de la composition témoin T1 non conforme à l'invention, ce qui indique un état de dispersion de la charge renforçante dans la composition C2 significativement amélioré par rapport à celui dans la composition témoin, donc une bonne interaction de l'élastomère greffé avec le composé D avec la charge renforçante. On constate également une amélioration des propriétés hystérétiques et des propriétés de renforcement de la composition C2 conforme à l'invention par rapport à celles de la composition T1, témoin non conforme.

De manière surprenante, la composition C2 qui comprend l'élastomère greffé avec le composé D selon l'invention présente des propriétés hystérétiques et des propriétés de renforcement significativement améliorées par rapport à la composition C1 non conforme à l'invention. Ces résultats sont surprenants car au vu des résultats de ΔG^{∗}_{à 60°C retour} de la composition C1 non conforme, on s'attendrait à ce que cette composition C1 présente de meilleures propriétés de renforcement par rapport à la composition C2 conforme à l'invention puisque la charge renforçante inorganique est mieux dispersée dans cette composition que dans la composition C2 (ΔG^{∗}_{à 60°C retour} de la composition C1 inférieur à ΔG^{∗}_{à 60°C retour} de la composition C2).

## Revendications

1. Composé de formule (I) 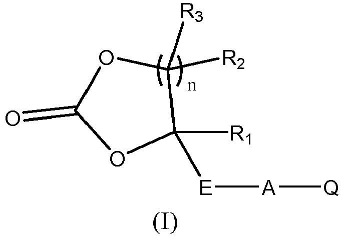 dans laquelle
- Q représente un dipôle comprenant au moins un atome d'azote ;
- A représente un cycle arènediyle, éventuellement substitué par une ou plusieurs chaînes hydrocarbonées, identiques ou différentes, indépendantes les unes des autres, éventuellement substituées ou interrompues par un ou plusieurs hétéroatomes ;
- E représente un groupe de liaison divalent hydrocarboné pouvant éventuellement contenir un ou plusieurs hétéroatomes ;
- R₁, R₂ et R₃ représentent, indépendamment les uns des autres, un atome d'hydrogène ou une chaîne hydrocarbonée éventuellement substituée ou interrompue par un ou plusieurs hétéroatomes ; et
- n est un entier égal à 1, 2, 3 ou 4.

2. Composé selon la revendication 1, dans lequel le groupement Q est un groupe de formule (II), (III) ou (IV) 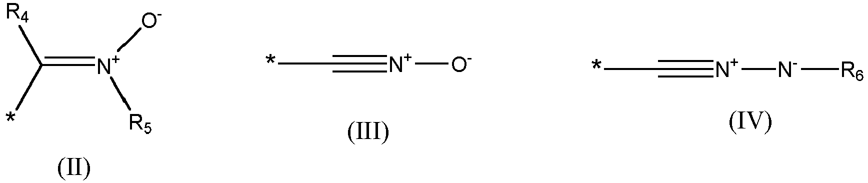 dans lesquelles :
- le symbole * représente le rattachement de Q à A ; et
- R₄, R₅ et R₆ sont choisis indépendamment parmi un atome d'hydrogène, un alkyle en C₁-C₂₀ linéaire ou ramifié, un cycloalkyle en C₃-C₃₀ éventuellement substitué par une chaîne hydrocarbonée, un aryle en C₆-C₂₀ éventuellement substitué par une chaîne hydrocarbonée.

3. Composé selon l'une quelconque des revendications précédentes, dans lequel le groupement A est un cycle arènediyle en C₆-C₁₄ éventuellement substitué par une ou plusieurs chaînes hydrocarbonées, identiques ou différentes, indépendantes les unes des autres, éventuellement substituées ou interrompues par un ou plusieurs hétéroatomes.

4. Composé selon l'une quelconque des revendications précédentes, dans lequel le composé de formule (I) est choisi dans le groupe formé par les composés de formule (Ia) et (Ib) 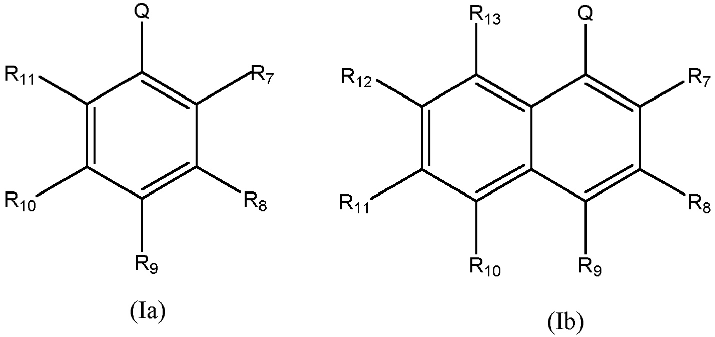 dans lesquelles :
- le groupement Q est tel que défini selon l'une quelconque des revendications 1 à 3 ;
- un groupement choisi parmi R₇ à R₁₁ de la formule (Ia) et un groupement choisi parmi R₇ à R₁₃ de la formule (Ib) désigne le groupe de formule (V) suivante : 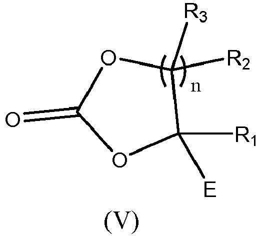 dans laquelle n, E, R₁, R₂ et R₃ sont tels que définis à la revendication 1 ; et
- les quatre autres groupements de la formule (Ia) et les six autres groupements de la formule (Ib), identiques ou différents, représentent indépendamment les uns les autres, un atome d'hydrogène ou une chaîne hydrocarbonée, linéaire ou ramifiée, de préférence saturée éventuellement substituée ou interrompue par un ou plusieurs hétéroatomes.

5. Composé selon l'une quelconque des revendications 2 à 4, dans lequel le composé de formule (I), dont le groupement Q est groupe de formule (III), est choisi parmi les composés de formule (VI) 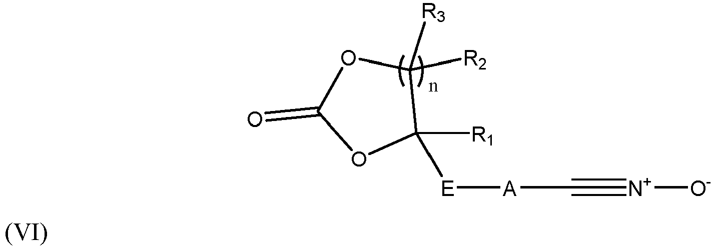 dans laquelle
- A est tel que définis à l'une quelconque des revendications 2 à 4 ;
- E représente un groupe de liaison divalent hydrocarboné pouvant éventuellement contenir un ou plusieurs hétéroatomes ;
- R₁, R₂ et R₃ représentent, indépendamment les uns des autres, un atome d'hydrogène ou une chaîne hydrocarbonée éventuellement substituée ou interrompue par un ou plusieurs hétéroatomes ; et
- n est un entier égal à 1, 2, 3 ou 4.

6. Composé selon l'une quelconque des revendications précédentes, dans lequel n=1 ou 2, plus préférentiellement n=1.

7. Composé selon l'une quelconque des revendications précédentes, dans lequel le groupement E est une chaîne hydrocarbonée linéaire ou ramifiée, de préférence saturée en C₁-C₂₄, préférentiellement en C₁-C₁₀, plus préférentiellement en C₁-C₆ éventuellement interrompue par un ou plusieurs atomes d'azote, de soufre ou d'oxygène.

8. Composé selon l'une quelconque des revendications précédentes, dans lequel groupement E est choisi dans le groupe constitué par -R- et -OR- où R est un alkylène, linéaire ou ramifié, en C₁-C₂₄, de préférence en C₁-C₁₀, plus préférentiellement en C₁-C₆.

9. Composé selon l'une quelconque des revendications précédentes, dans lequel le groupement E est choisi parmi -CH₂-, -CH₂-CH₂-, -CH₂-CH₂-CH₂-, -CH₂-CH₂-CH₂-CH₂-, -O-CH₂-, -O-CH₂-CH₂-, -O-CH₂-CH₂-CH₂- et -O-CH₂-CH₂-CH₂-CH₂-

10. Composé selon l'une quelconque des revendications précédentes, dans lequel les groupes R₁, R₂, R₃ représentent, indépendamment les uns des autres, un atome d'hydrogène ou un alkyle linéaire ou ramifié en C₁-C₂₄, préférentiellement en C₁-C₁₀, plus préférentiellement en C₁-C₆.

11. Composé selon l'une quelconque des revendications précédentes, dans lequel R₁, R₂ et R₃ sont un atome d'hydrogène.

12. Composé selon l'une quelconque des revendications 5 à 11, dans lequel le composé de formule (VI) est choisi dans le groupe constitué par le composé de formule (VII) et le composé de formule (VIII)

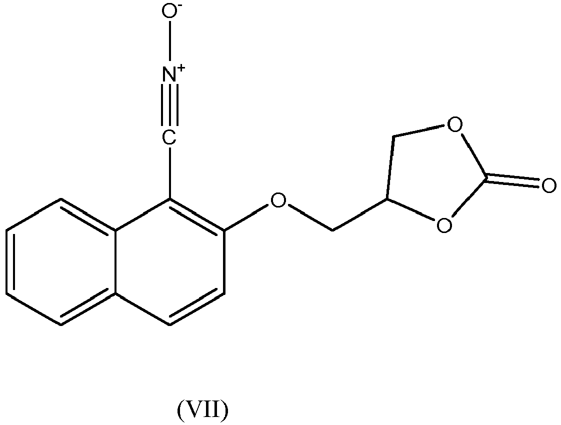

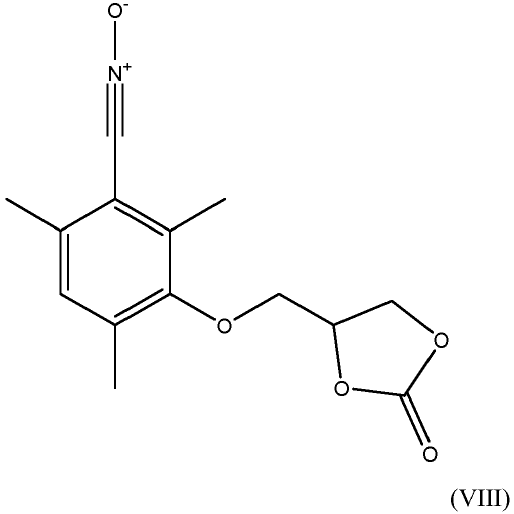

13. Procédé de de préparation d'un composé de formule (VI) comprenant au moins une réaction d'un composé oxime de formule (IX) avec un agent oxydant en présence d'au moins un solvant organique SL1 selon le schéma réactionnel suivant : 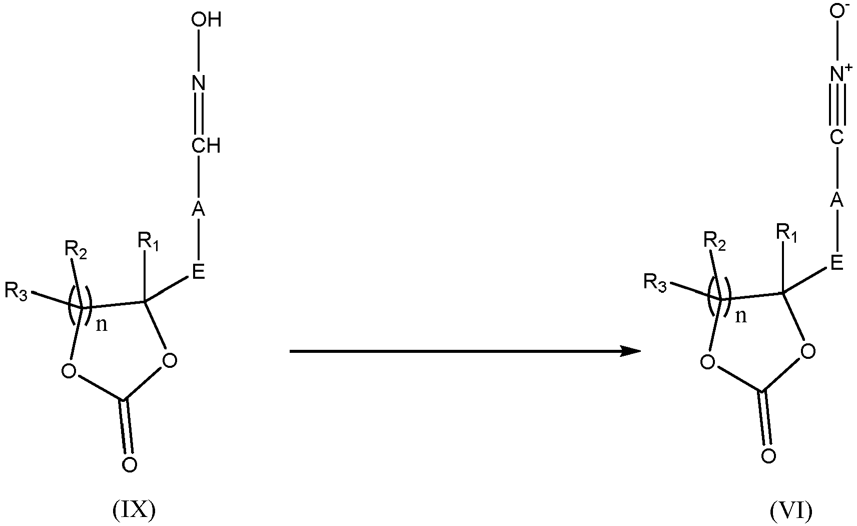 avec :
- A représente un cycle arènediyle, éventuellement substitué par une ou plusieurs chaînes carbonées, identiques ou différentes, indépendantes les unes des autres, éventuellement substituées ou interrompues par un ou plusieurs hétéroatomes ;
- E représente un groupe divalent hydrocarboné pouvant éventuellement contenir un ou plusieurs hétéroatomes ;
- R₁, R₂ et R₃ représentent, indépendamment les uns des autres, un atome d'hydrogène ou une chaîne hydrocarbonée éventuellement substituée ou interrompue par un ou plusieurs hétéroatomes ; et
- n est un entier égal à 1, 2, 3 ou 4.

14. Procédé de préparation d'un composé oxime de formule (IX) comprenant au moins une réaction d'un composé de formule (X) avec une solution aqueuse d'hydroxylamine (IX) selon le schéma réactionnel suivant : 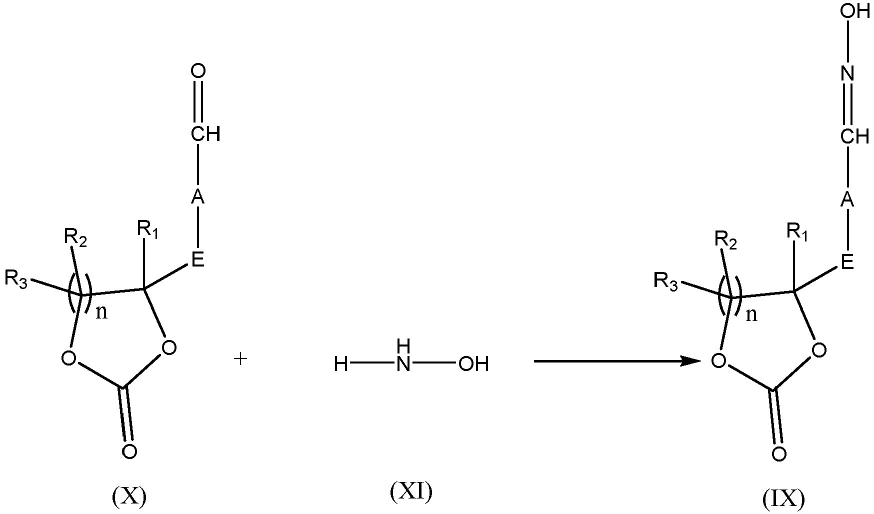 avec :
- A représente un cycle arènediyle, éventuellement substitué par une ou plusieurs chaînes carbonées, identiques ou différentes, indépendantes les unes des autres, éventuellement substituées ou interrompues par un ou plusieurs hétéroatomes ;
- E représente un groupe divalent hydrocarboné pouvant éventuellement contenir un ou plusieurs hétéroatomes ;
- R₁, R₂ et R₃ représentent, indépendamment les uns des autres, un atome d'hydrogène ou une chaîne hydrocarbonée éventuellement substituée ou interrompue par un ou plusieurs hétéroatomes ; et
- n est un entier égal à 1, 2, 3 ou 4.

15. Procédé selon la revendication 14, dans lequel l'ajout d'hydroxylamine (XI) est effectué en deux fois.

## Patentansprüche

1. Verbindung der Formel (I) 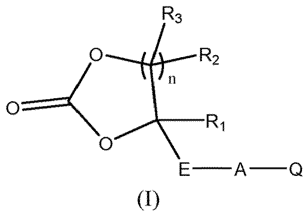 in der:
- Q für einen Dipol mit mindestens einem Stickstoffatom steht;
- A für einen Arendiylring steht, der gegebenenfalls durch eine oder mehrere gleiche oder verschiedene, voneinander unabhängige Kohlenwasserstoffketten, die gegebenenfalls durch ein oder mehrere Heteroatome substituiert oder unterbrochen sind, substituiert ist;
- E für eine zweiwertige Kohlenwasserstoffverknüpfungsgruppe steht, die gegebenenfalls ein oder mehrere Heteroatome enthalten kann;
- R₁, R₂ und R₃ unabhängig voneinander für ein Wasserstoffatom oder eine Kohlenwasserstoffkette, die gegebenenfalls durch ein oder mehrere Heteroatome substituiert oder unterbrochen ist, stehen; und
- n eine ganze Zahl mit einem Wert von 1, 2, 3 oder 4 ist.

2. Verbindung nach Anspruch 1, wobei es sich bei der Gruppe Q um eine Gruppe der Formel (II), (III) oder (IV) handelt: 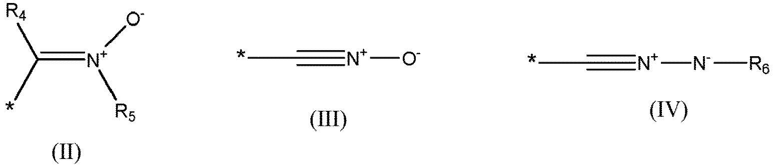 in denen:
- das Symbol * für die Anbindung von Q an A steht; und
- R₄, R₅ und R₆ unabhängig voneinander aus einem Wasserstoffatom, einem linearen oder verzweigten C₁-C₂₀-Alkyl, einem C₃-C₃₀-Cycloalkyl, das gegebenenfalls durch eine Kohlenwasserstoffkette substituiert ist, und einem C₆-C₂₀-Aryl, das gegebenenfalls durch eine Kohlenwasserstoffkette substituiert ist, ausgewählt sind.

3. Verbindung nach einem der vorhergehenden Ansprüche, wobei es sich bei der Gruppe A um einen C₆-C₁₄-Arendiylring handelt, der gegebenenfalls durch eine oder mehrere gleiche oder verschiedene, voneinander unabhängige Kohlenwasserstoffketten, die gegebenenfalls durch ein oder mehrere Heteroatome substituiert oder unterbrochen sind, substituiert ist.

4. Verbindung nach einem der vorhergehenden Ansprüche, wobei die Verbindung der Formel (I) aus der durch die Verbindungen der Formel (Ia) und (Ib) gebildeten Gruppe ausgewählt ist: 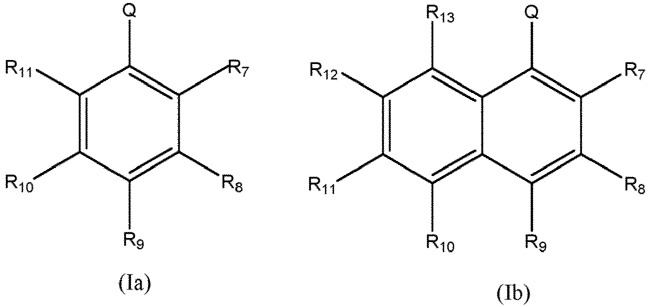 in denen:
- die Gruppe Q wie gemäß einem der Ansprüche 1 bis 3 definiert ist;
- eine aus R₇ bis R₁₁ der Formel (Ia) ausgewählte Gruppe und eine aus R₇ bis R₁₃ der Formel (Ib) ausgewählte Gruppe die folgende Gruppe (V) bedeutet: 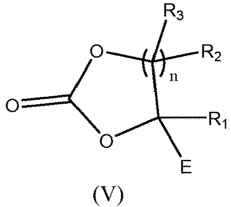 in der n, E, R₁, R₂ und R₃ wie in Anspruch 1 definiert sind; und
- die vier anderen Gruppen der Formel (Ia) und die sechs anderen Gruppen der Formel (Ib) gleich oder verschieden sind und unabhängig voneinander für ein Wasserstoffatom oder eine lineare oder verzweigte, vorzugsweise gesättigte Kohlenwasserstoffkette, die gegebenenfalls durch ein oder mehrere Heteroatome substituiert oder unterbrochen ist, stehen.

5. Verbindung nach einem der Ansprüche 2 bis 4, wobei die Verbindung der Formel (I), in der es sich bei der Gruppe Q um die Gruppe der Formel (III) handelt, aus den Verbindungen der Formel (VI) ausgewählt ist: 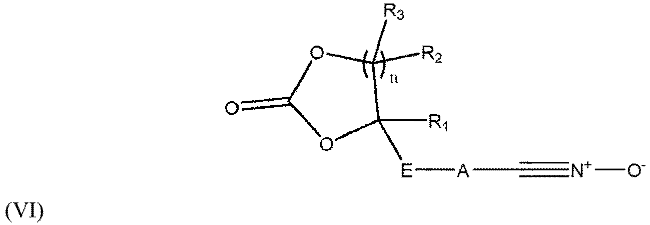 in der
- A wie in einem der Ansprüche 2 bis 4 definiert ist;
- E für eine zweiwertige Kohlenwasserstoffverknüpfungsgruppe steht, die gegebenenfalls ein oder mehrere Heteroatome enthalten kann;
- R₁, R₂ und R₃ unabhängig voneinander für ein Wasserstoffatom oder eine Kohlenwasserstoffkette, die gegebenenfalls durch ein oder mehrere Heteroatome substituiert oder unterbrochen ist, stehen; und
- n eine ganze Zahl mit einem Wert von 1, 2, 3 oder 4 ist.

6. Verbindung nach einem der vorhergehenden Ansprüche, wobei n = 1 oder 2, weiter bevorzugt n = 1.

7. Verbindung nach einem der vorhergehenden Ansprüche, wobei es sich bei der Gruppe E um eine lineare oder verzweigte, vorzugsweise gesättigte C₁-C₂₄-, vorzugsweise C₁-C₁₀- und weiter bevorzugt C₁-C₆-Kohlenwasserstoffkette, die gegebenenfalls durch ein oder mehrere Stickstoff-, Schwefel- oder Sauerstoffatome unterbrochen ist, handelt.

8. Verbindung nach einem der vorhergehenden Ansprüche, wobei die Gruppe E aus der Gruppe bestehend aus -R- und -OR- ausgewählt ist, wobei R für ein lineares oder verzweigtes C₁-C₂₄-, vorzugsweise C₁-C₁₀- und weiter bevorzugt C₁-C₆-Alkylen steht.

9. Verbindung nach einem der vorhergehenden Ansprüche, wobei die Gruppe E aus -CH₂-, -CH₂-CH₂-, -CH₂-CH₂-CH₂-, - CH₂-CH₂-CH₂-CH₂-, -O-CH₂-, -O-CH₂-CH₂-, -O-CH₂-CH₂-CH₂- und -O-CH₂-CH₂-CH₂-CH₂- ausgewählt ist.

10. Verbindung nach einem der vorhergehenden Ansprüche, wobei die Gruppen R₁, R₂ und R₃ unabhängig voneinander für ein Wasserstoffatom oder ein lineares oder verzweigtes C₁-C₂₄-, vorzugsweise C₁-C₁₀- und weiter bevorzugt C₁-C₆-Alkyl stehen.

11. Verbindung nach einem der vorhergehenden Ansprüche, wobei es sich bei R₁, R₂ und R₃ um ein Wasserstoffatom handelt.

12. Verbindung nach einem der Ansprüche 5 bis 11, wobei die Verbindung der Formel (VI) aus der Gruppe bestehend aus der Verbindung der Formel (VII) und der Verbindung der Formel (VIII) ausgewählt ist:

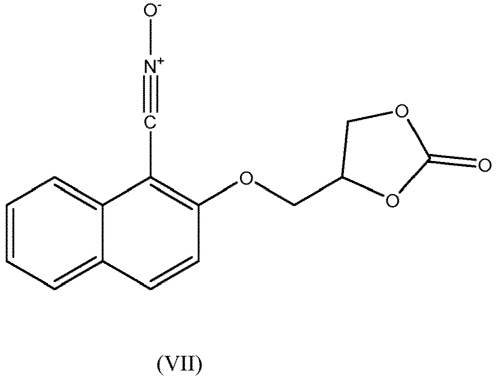

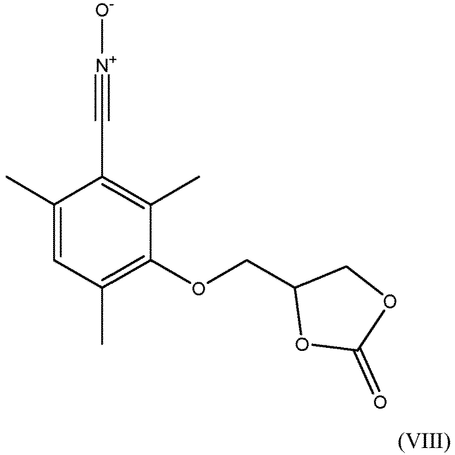

13. Verfahren zur Herstellung einer Verbindung der Formel (VI), umfassend mindestens eine Umsetzung einer Oximverbindung der Formel (IX) mit einem Oxidationsmittel in Gegenwart mindestens eines organischen Lösungsmittels SL1 gemäß dem folgenden Reaktionsschema: 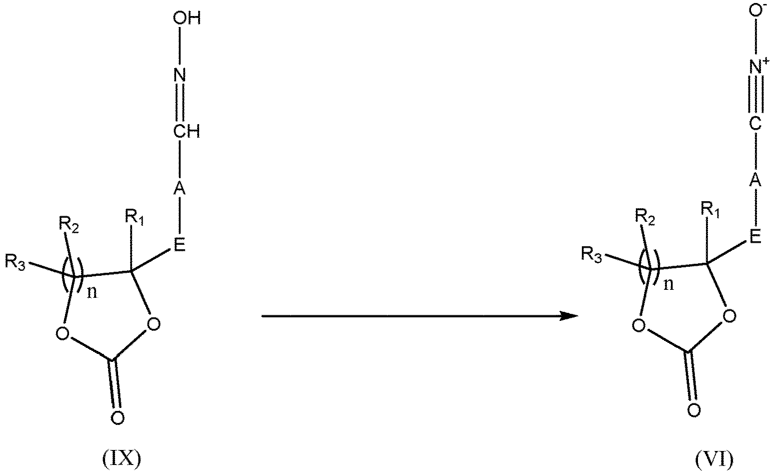 wobei:
- A für einen Arendiylring steht, der gegebenenfalls durch eine oder mehrere gleiche oder verschiedene, voneinander unabhängige Kohlenstoffketten, die gegebenenfalls durch ein oder mehrere Heteroatome substituiert oder unterbrochen sind, substituiert ist;
- E für eine zweiwertige Kohlenwasserstoffgruppe steht, die gegebenenfalls ein oder mehrere Heteroatome enthalten kann;
- R₁, R₂ und R₃ unabhängig voneinander für ein Wasserstoffatom oder eine Kohlenwasserstoffkette, die gegebenenfalls durch ein oder mehrere Heteroatome substituiert oder unterbrochen ist, stehen; und
- n eine ganze Zahl mit einem Wert von 1, 2, 3 oder 4 ist.

14. Verfahren zur Herstellung einer Oximverbindung der Formel (IX), umfassend mindestens eine Umsetzung einer Verbindung der Formel (X) mit einer wässrigen Lösung von Hydroxylamin (IX) gemäß dem folgenden Reaktionsschema: 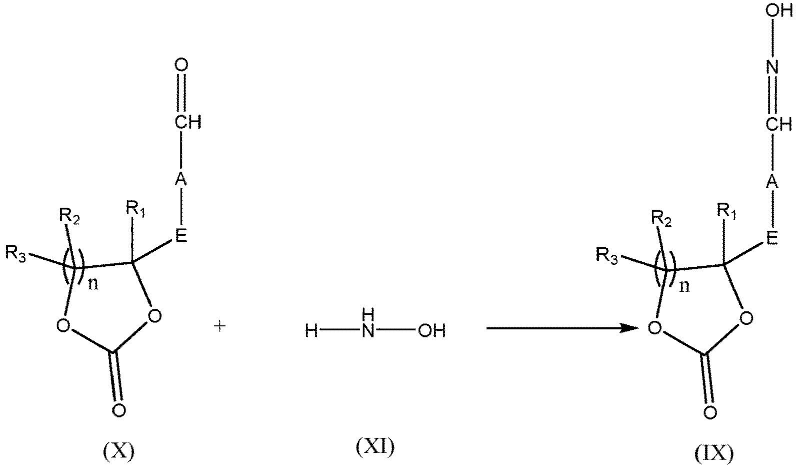 wobei:
- A für einen Arendiylring steht, der gegebenenfalls durch eine oder mehrere gleiche oder verschiedene, voneinander unabhängige Kohlenstoffketten, die gegebenenfalls durch ein oder mehrere Heteroatome substituiert oder unterbrochen sind, substituiert ist;
- E für eine zweiwertige Kohlenwasserstoffgruppe steht, die gegebenenfalls ein oder mehrere Heteroatome enthalten kann;
- R₁, R₂ und R₃ unabhängig voneinander für ein Wasserstoffatom oder eine Kohlenwasserstoffkette, die gegebenenfalls durch ein oder mehrere Heteroatome substituiert oder unterbrochen ist, stehen; und
- n eine ganze Zahl mit einem Wert von 1, 2, 3 oder 4 ist.

15. Verfahren nach Anspruch 14, wobei die Zugabe von Hydroxylamin (XI) in zwei Stufen durchgeführt wird.

## Claims

1. Compound of formula (I) 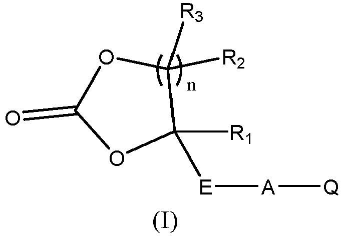 in which:
- Q represents a dipole comprising at least one nitrogen atom;
- A represents an arenediyl ring, optionally substituted by one or more hydrocarbon-based chains, which are identical or different, independent of one another, and are optionally substituted or interrupted by one or more heteroatoms;
- E represents a divalent hydrocarbon-based bonding group which may optionally contain one or more heteroatoms;
- R₁, R₂ and R₃ represent, independently of one another, a hydrogen atom or a hydrocarbon-based chain which is optionally substituted or interrupted by one or more heteroatoms; and
- n is an integer equal to 1, 2, 3 or 4.

2. Compound according to Claim 1, in which the group Q is a group of formula (II), (III) or (IV) 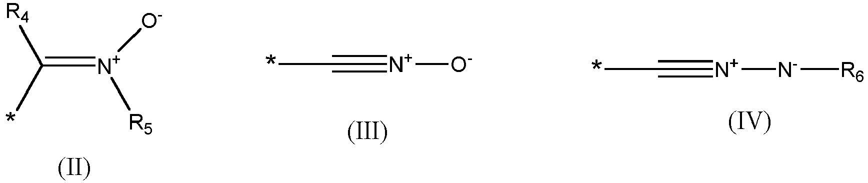 in which:
- the symbol * represents the attachment of Q to A; and
- R₄, R₅ and R₆ are independently selected from a hydrogen atom, a linear or branched C₁-C₂₀ alkyl, a C₃-C₃₀ cycloalkyl which is optionally substituted by a hydrocarbon-based chain, and a C₆-C₂₀ aryl which is optionally substituted by a hydrocarbon-based chain.

3. Compound according to any one of the preceding claims, in which the group A is a C₆-C₁₄ arenediyl ring optionally substituted by one or more hydrocarbon-based chains, which are identical or different, independent of one another, and are optionally substituted or interrupted by one or more heteroatoms.

4. Compound according to any one of the preceding claims, in which the compound of formula (I) is selected from the compounds of formulae (Ia) and (Ib) 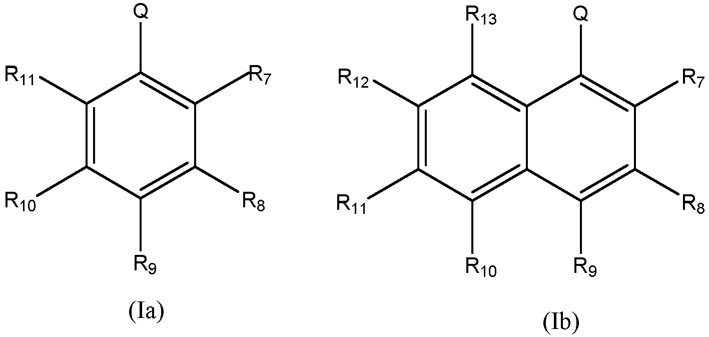 in which:
- the group Q is as defined according to any one of Claims 1 to 3;
- one group selected from R₇ to R₁₁ of the formula (Ia) and one group selected from R₇ to R₁₃ of the formula (Ib) denotes the following group of formula (V): 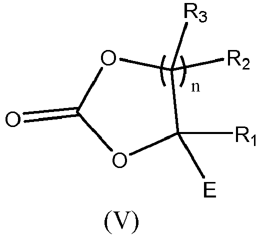 in which
- n, E, R₁, R₂ and R₃ are as defined in Claim 1,
- the four other groups of the formula (Ia) and the six other groups of the formula (Ib), which are identical or different, independently of one another represent a hydrogen atom or a linear or branched, preferably saturated hydrocarbon-based chain which is optionally substituted or interrupted by one or more heteroatoms.

5. Compound according to any one of Claims 2 to 4, in which the compound of formula (I), in which the group Q is a group of formula (III), is selected from the compounds of formula (VI) 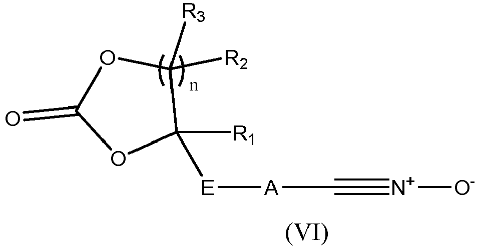 in which
- A is as defined in any one of Claims 2 to 4,
- E represents a divalent hydrocarbon-based bonding group which may optionally contain one or more heteroatoms;
- R₁, R₂ and R₃ represent, independently of one another, a hydrogen atom or a hydrocarbon-based chain which is optionally substituted or interrupted by one or more heteroatoms; and
- n is an integer equal to 1, 2, 3 or 4.

6. Compound according to any one of the preceding claims, in which n = 1 or 2, more preferentially n = 1.

7. Compound according to any one of the preceding claims, in which the group E is a linear or branched, preferably saturated, C₁-C₂₄, more preferentially C₁-C₁₀, even more preferentially C₁-C₆, hydrocarbon-based chain which is optionally interrupted by one or more nitrogen, sulfur or oxygen atoms

8. Compound according to any one of the preceding claims, in which the group E is selected from the group consisting of -R- and -OR-, where R is a linear or branched C₁-C₂₄, preferably C₁-C₁₀, more preferentially C₁-C₆, alkylene.

9. Compound to any one of the preceding claims, in which the group E is selected from -CH₂-, -CH₂-CH₂-, -CH₂-CH₂-CH₂-, -CH₂-CH₂-CH₂-CH₂-, -O-CH₂-, -O-CH₂-CH₂-, -O-CH₂-CH₂-CH₂- and -O-CH₂-CH₂-CH₂-CH₂-.

10. Compound according to any one of the preceding claims, in which the groups R₁, R₂ and R₃ represent, independently of one another, a hydrogen atom or a linear or branched C₁-C₂₄, preferentially C₁-C₁₀, more preferentially C₁-C₆, alkyl.

11. Compound according to any one of the preceding claims, in which the groups R₁, R₂ and R₃ are a hydrogen atom.

12. Compound according to any one of the Claims 5 to 11, in which the compound of formula (I) is selected from the group consisting of the compound of formula (VII) and the compound of formula (VIII)

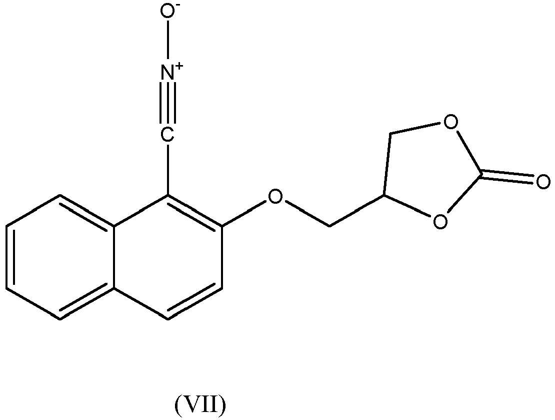

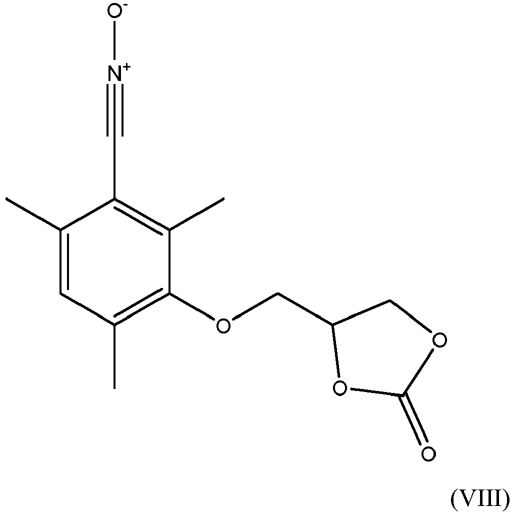

13. Process for preparing a compound of formula (I) comprising at least a reaction of an oxime compound of formula (IX) with an oxidizing agent in the presence of at least one organic solvent SL1 according to the following reaction scheme: 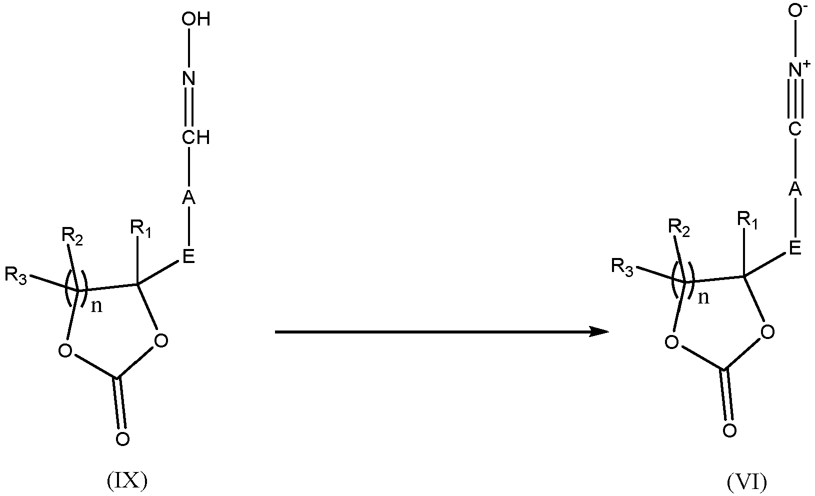 where:
- A represents an arenediyl ring, optionally substituted by one or more carbon-based chains, which are identical or different, independent of one another, and are optionally substituted or interrupted by one or more heteroatoms;
- E represents a divalent hydrocarbon-based group which may optionally contain one or more heteroatoms;
- R₁, R₂ and R₃ represent, independently of one another, a hydrogen atom or a hydrocarbon-based chain which is optionally substituted or interrupted by one or more heteroatoms; and
- n is an integer equal to 1, 2, 3 or 4.

14. Process for preparing a oxime compound of formula (IX) comprising at least one reaction of a compound of formula (X) with an aqueous solution of hydroxylamine NHzOH (XI) according to the following reaction scheme: 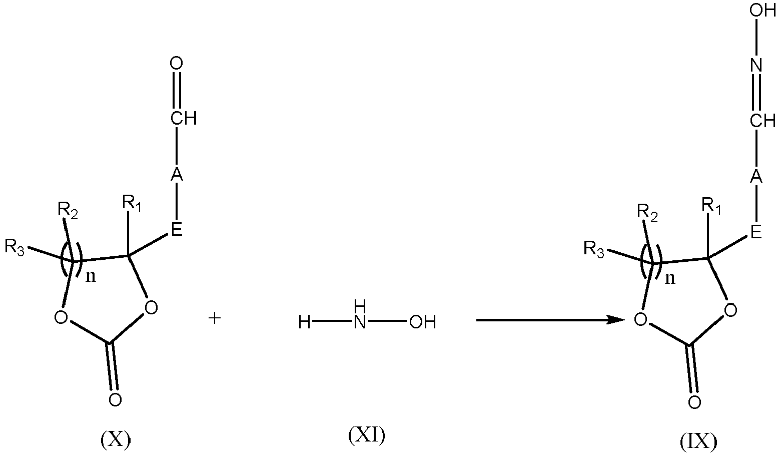 where:
- A represents an arenediyl ring, optionally substituted by one or more carbon-based chains, which are identical or different, independent of one another, and are optionally substituted or interrupted by one or more heteroatoms;
- E represents a divalent hydrocarbon-based group which may optionally contain one or more heteroatoms;
- R₁, R₂ and R₃ represent, independently of one another, a hydrogen atom or a hydrocarbon-based chain which is optionally substituted or interrupted by one or more heteroatoms; and
- n is an integer equal to 1, 2, 3 or 4.

15. Process according to Claim 14, wherein the addition of hydroxylamine is carried out in two stages.
